# EUROPÄISCHE PATENTSCHRIFT

(11) **EP 1 648 916 B1**
(45) Veröffentlichungstag und Bekanntmachung des Hinweises auf die Patenterteilung: **08.10.2008**
(21) Anmeldenummer: 04766209.3
(22) Anmeldetag: 13.07.2004
(51) Int. Cl.: C07J 75/00

(54) **VERFAHREN ZUR GEWINNUNG EINES NATÜRLICHEN GEMISCHES KONJUGIERTER EQUIN ESRTROGENE**
METHOD FOR OBTAINING A NATURAL MIXTURE OF CONJUGATED EQUINE ESTROGENS
PROCEDE D'OBTENTION D'UN MELANGE NATUREL D'OESTROGENES EQUINS CONJUGUES

(30) Priorität: 17.07.2003 EP 03102215
(43) Veröffentlichungstag der Anmeldung: 26.04.2006
(73) Patentinhaber: Solvay Pharmaceuticals GmbH, 30173 Hannover (DE)
(72) Erfinder: RASCHE, Heinz-Helmer, 31303 Burgdorf (DE); WILBRAND, Kirsten, 31515 Wunstorf (DE); BANSCHBACH, Sabine, 29690 Schwarmstedt (DE)
(74) Vertreter: Gosmann, Martin
(86) Internationale Anmeldenummer: PCT/EP2004/051479
(87) Internationale Veröffentlichungsnummer: WO 2005/010021

(56) Entgegenhaltungen:
- WO-A-98/08526
- WO-A-03/048183

## Beschreibung

Die vorliegende Erfindung betrifft die Gewinnung eines natürlichen Gemisches konjugierter equiner Östrogene, welches an phenolischen Harninhaltsstoffen und an nicht-konjugierten lipophilen Verbindungen aus der Gruppe umfassend nicht-konjugierte Flavonoide, nicht-konjugierte Isoflavonoide, nicht-konjugierte Norisoprenoide, nicht-konjugierte Steroide, insbesondere Androstan- und Pregnan-Steroide, und vergleichbare nicht-konjugierte Verbindungen, abgereichert ist.

Östrogene werden in der Medizin zur Hormonsubstitutionstherapie eingesetzt. Insbesondere werden Östrogengemische zur Behandlung und Prophylaxe der, bei Frauen auftretenden Beschwerden der Wechseljahre nach natürlicher oder artifizieller Menopause eingesetzt. Hierbei haben sich natürliche Gemische konjugierter Östrogene, wie sie im Harn trächtiger Stuten vorliegen, im Folgenden als natürliche Gemische konjugierter equiner Östrogene bezeichnet, als besonders wirksam und gut verträglich erwiesen.

Der gelöste Feststoffgehalt im Harn trächtiger Stuten (= pregnant mares urine, im Folgenden abgekürzt als "PMU") kann natürlicherweise in weiten Bereichen schwanken und im Allgemeinen in einem Bereich von 40 bis 90 g Trockensubstanz pro Liter liegen. Neben Harnstoff und sonstigen üblichen Harninhaltsstoffen sind in dem Feststoffgehalt des PMU phenolische Bestandteile in Mengen von ca. 2 bis 5 Gew.-% bezogen auf die Trockensubstanz enthalten. Unter diesen phenolischen Bestandteilen befinden sich Kresole und das als HPMF bekannte Dihydro-3,4-bis[(3-hydroxyphenyl)methyl]-2(3H)-furanon. Diese können in freier oder konjugierter Form vorliegen. In dem PMU ist ein natürliches Gemisch von Östrogenen enthalten, welches weitgehend in konjugierter Form, z. B. als Schwefelsäurehalbester-Natriumsalz (im Folgenden abgekürzt als "Sulfatsalz"), vorliegt. Der Gehalt an konjugierten Östrogenen (berechnet als Östrogensulfatsalz) kann zwischen 0,1 und 0,5 Gew.-% bezogen auf die Trockensubstanz betragen. Zusätzlich können im Feststoffgehalt des PMU weitere lipophile Verbindungen vorliegen, deren Mengen in weiten Bereichen schwanken können und nicht vorhersehbar sind. Diese lipophilen Verbindungen stammen überwiegend aus den von den trächtigen Stuten als Nahrung aufgenommen Pflanzen und umfassen vor allem verschiedene Flavonoid-, Isoflavonoid-, Nor-isoprenoid-Derivate und vergleichbare Verbindungen, wie beispielsweise Formononetin, Genistein, Daidzein, Biochanin A, Equol und Coumestrol. Diese lipophilen Verbindungen ursprünglich pflanzlicher Herkunft können im Harn in konjugierter oder in freier (nicht konjugierter) Form vorliegen. Zu den, im Feststoffgehalt des PMU weiterhin vorkommenden lipophilen Bestandteilen zählen auch nicht-konjugierte Steroid-Derivate, hier seinen insbesondere die Androstan- und Pregnan-Steroide, aber auch nicht-konjugierte Östrogen-Derivate genannt.

Natürliche Gemische konjugierter Östrogene enthaltende Extrakte werden üblicherweise entweder mittels eines Festphasen-Extraktionsverfahren oder durch ein Verfahren gewonnen, das auf verschiedenen Flüssig-Flüssig-Extraktionsschritten mit, mit Wasser nicht oder nur wenig mischbaren organischen Lösemitteln basiert. Allgemein gilt, dass das gewonnene natürliche Gemisch konjugierter Östrogene, um als Wirkstoffkomponente für Pharmazeutika verwendet werden zu können, bestimmte pharmazeutische Spezifikationen erfüllen muss, beispielsweise muss es der in der USP (United States Pharmacopeia) oder European Pharmacopeia festgelegten Spezifikation entsprechen. So müssen hinsichtlich des Gehalts an konjugierten Östrogenen bezogen auf die Trockensubstanz bestimmte Grenzwerte eingehalten werden.

Die US Patente No. 2,551,205 und No. 2,429,398 beschreiben ein Verfahren zur Herstellung einer wasser-löslichen Östrogen-Präparation aus PMU, bei dem zunächst durch Adsorption an Aktivkohle oder andere geeignete Adsorbermaterialien, Elution mit einem mit Wasser mischbaren organischen Lösungsmittel, wie z. B. Pyridin, und anschließende Entfernung des Lösungsmittels ein wässriges Konzentrat erhalten wird, welches den größten Teil der wasserlöslichen Östrogen-Bestandteile des ursprünglich eingesetzten PMUs enthält. Während im US Patent No. 2,429,398 das Konzentrat durch Extraktion mit Benzol und / oder Ether weiter aufgereinigt wird, wird im US Patent No. 2,551,205 beschrieben, dass das Konzentrat auf einen pH-Wert zwischen 2 und 6, vorzugsweise zwischen 4 und 5, angesäuert und dann schnell mit einem mit Wasser kaum mischbaren organischen Lösungsmittel aus der Gruppe der aliphatischen, aromatischen oder alizyklischen Kohlenwasserstoffe (z. B. Hexan, Benzol, Toluol, Cyclohexan) oder der chlorierten Kohlenwasserstoffe (z. B. Chloroform, Ethylendichlorid, Trichlorethylen, Tetrachlorkohlenstoff, Chlorbenzol) extrahiert wird, um unerwünschte Substanzen wie Fette, Öle, freie phenolische Bestandteile und die nicht-konjugierten Steroide durch Überführung in die organische Phase abzutrennen. Abschließend wird die wässrige Phase durch Neutralisation stabilisiert. Das US Patent No. 2,551, 205 empfiehlt, den gewonnenen Extrakt durch nachfolgende Extraktionsschritte und Präzipitationen weiter aufzureinigen. Insgesamt wird nach Durchführung des, im US Patent No. 2,551,205 beschriebenen Verfahrens nur eine Ausbeute von etwa 80% der Östrogen-Bestandteile des eingesetzten Konzentrates erhalten.

Im US Patent No. 2,565,115 wird die Extraktion der konjugierten Östrogene aus PMU mit Aceton beschrieben. Über die Reinheit der erhaltenen Östrogenfraktion wird keine Aussage gemacht.

Das US Patent No. 2,696,265 beschreibt ein Verfahren, in dem zunächst die Östrogene mit einem aliphatischen Alkohol oder Keton, wie Hexanol, Cyclohexanol oder Cyclohexanon, extrahiert werden. Die Östrogene treten in die organische Phase über und werden anschließend weiter aufgereinigt, u.a. wird eine die Östrogene enthaltende wässrige Phase mit Salzsäure auf einen pH-Wert von 4 eingestellt und mit Ethylendichlorid extrahiert.

Im US Patent Nr. 2,834,712 wird ein Verfahren zur Herstellung von Östrogengemischen signifikanter Reinheit und geringer Toxizität offenbart, das auf einer Vielzahl einzelner Extraktionsschritte mit unterschiedlichen Lösungsmitteln und bei Einstellung verschiedener pH-Werte beruht. Dabei finden große Volumina an Lösungsmitteln wie Hexan und Benzol Verwendung. So wird z. B. in einem Schritt ein bereits aufgereinigtes Konzentrat in Wasser gelöst, mit Salzsäure auf einen pH-Wert von circa 5.0 eingestellt und mit Benzol und anschließend mit Ether extrahiert, um die phenolischen Bestandteile abzutrennen.

Die internationale Patentanmeldung WO 01/27134 beschreibt ein vergleichsweise einfaches Verfahren, um konjugierte Östrogene aus PMU zu extrahieren: Nach Zugabe eines Salzes, wie z. B. Natriumchlorid, wird der PMU mit mindestens dem gleichen Volumenanteil eines organischen Lösungsmittels, wie z. B. Ethylacetat, extrahiert, dabei treten die konjugierten Östrogene in die organische Phase über. Die organische Phase wird abgetrennt und getrocknet, um den Extrakt zu erhalten. In der WO 01/27134 werden keine Aussagen über die Reinheit des erhaltenen Extraktes konjugierter Östrogene gemacht.

Bei den vorstehend beschriebenen und aus dem Stand der Technik bekannten Flüssig-Flüssig-Extraktionsverfahren treten jedoch eine Vielzahl von Problemen, wie starke Schaumbildung, Sedimentbildung, Emulsionsbildung und schlechte Phasentrennung auf. Es werden im Allgemeinen mehrere Extraktionsschritte benötigt, was zu Verlusten und nur teilweisen Gewinnung des Östrogengehaltes führt. Weiterhin erfordern diese Extraktionsverfahren große Volumina an zum Teil gesundheitsschädlichen Lösungsmitteln. Weiterhin werden in den oben angeführten Patentschriften weder über den Gehalt an nicht-konjugierten lipophilen Bestandteilen, wie beispielsweise nicht-konjugierte Flavonoid-, Isoflavonoid-, Norisoprenoid-Derivate und vergleichbare nicht-konjugierte Verbindungen, oder auch nicht-konjugierte Steroide, insbesondere Androstan- und Pregnan-Steroide, in den erhaltenen Produkten noch über eine Abtrennung dieser Bestandteile Aussagen gemacht. Diese aus dem Stand der Technik bekannten Verfahren liefern entweder keine zufriedenstellenden Ergebnisse hinsichtlich der Ausbeute oder hinsichtlich der Reinheit des erhaltenen Extraktes, gemessen am erhaltenen Gesamthormongehalt bezogen auf die Trockensubstanz, oder sie beruhen auf einer Vielzahl verschiedener Verfahrensschritte und der Verwendung großer Volumina organischer und z. T. sogar aus toxikologischer Sicht unerwünschter Lösungsmittel.

Weiterhin sind aus dem Stand der Technik verschiedene Festphasen-Extraktionsverfahren bekannt, um ein, an phenolischen Harninhaltsstoffen weitgehend abgereichertes, natürliches Gemisch konjugierter equiner Östrogene zu gewinnen.

So beschreibt die internationale Patentanmeldung WO 98/08526 ein Verfahren, mit welchem in einer Festphasen-Extraktion an einem semipolaren, insbesondere an einem nichtionischen semipolaren, polymeren Adsorberharz ein, an phenolischen Harninhaltsstoffen abgereichertes, weitgehend Kresol- und HPMF-freies, den natürlichen Östrogen-Gehalt des PMU praktisch vollständig enthaltendes Gemisch gewonnen werden kann. In der internationalen Patentanmeldung WO 98/08526 wird ein ähnliches Verfahren beschrieben, in dem bei der Festphasen-Extraktion Kieselgel als Adsorbermaterial zum Einsatz kommt. Auch die chinesische Patentanmeldung CN 1308083 beschreibt ein vergleichbares Verfahren, bei dem Cyanogruppen-haltige polare Adsorberharze verwendet werden. Weiterhin wird in der US Patentanmeldung US 2002/0156303 ein Verfahren beschrieben, bei dem der PMU vor der Aufreinigung über ein Polystyrol Adsorberharz zunächst mit einem alkalischen Solvenz behandelt und durch Filtration vorgereinigt wird. Die so erhaltenen Extrakte eignen sich als Ausgangsmaterial für die Herstellung von Pharmazeutika, welche als Wirkstoffkomponente das natürliche Gemisch konjugierter Östrogene aus PMU enthalten.

Die gestellten pharmazeutischen Spezifikationsanforderungen, beispielsweise die hinsichtlich des Gehalts an konjugierten Östrogenen bezogen auf die Trockensubstanz einzuhaltenden Grenzwerte, werden normalerweise von den aus PMU gemäß dem Verfahren der WO 98/08526 oder dem Verfahren der WO 98/08525, gewonnenen Gemischen konjugierter Östrogene erfüllt. Insbesondere kann mit Hilfe der dort offenbarten Verfahren ein an phenolischen Harninhaltsstoffen abgereichertes, weitgehend Kresol- und HPMF-freies Produkt gewonnen werden. Es hat sich aber herausgestellt, dass neben dem gewünschten Gehalt an konjugierten Östrogenen auch nicht-konjugierte lipophile Verbindungen in der gewonnenen Trockensubstanz enthalten sein können. Zu den nicht-konjugierten lipophilen Verbindungen zählen beispielsweise verschiedene nicht-konjugierte Flavonoid-, Isoflavonoid-, Norisoprenoid-Derivate und vergleichbare nicht-konjugierte Verbindungen, wie beispielsweise Formononetin, Genistein, Daidzein, Biochanin A, Equol und Coumestrol, aber auch nicht-konjugierte Steroide, insbesondere Androstan- und Pregnan-Steroide, und nicht-konjugierte Östrogene, wobei diese Aufzählung nicht als abschließend zu betrachten ist. Die Anwesenheit dieser nicht-konjugierten lipophilen Verbindungen im aus dem PMU gewonnenen Gemisch konjugierter Östrogene lässt sich nicht standardisieren, sondern sowohl der Gehalt als auch die Zusammensetzung der freien und der konjugierten lipophilen Verbindungen variiert beispielsweise in Abhängigkeit von der aufgenommenen Nahrung der trächtigen Stuten.

Zwar verändert sich die Zusammensetzung des natürlichen Gemisches an konjugierten equinen Östrogenen durch die zusätzliche Anwesenheit der nicht-konjugierten lipophilen Verbindungen nicht, aber der Gehalt der konjugierten equinen Östrogene bezogen auf die Trockensubstanz kann vermindert sein. Durch eine gezielte Abtrennung der nicht-konjugierten lipophilen Bestandteile könnte eine höhere Konzentration der Wirksubstanzen, d.h. der konjugierten equinen Östrogenen, im erhaltenen Extrakt erreicht werden. Weiterhin gewährleistet eine Abtrennung der nicht-konjugierten lipophilen Verbindungen eine gleichmäßigere Zusammensetzung einzelner Extraktchargen, da so die nicht-konjugierten lipophilen Bestandteile, deren Gehalt und Zusammensetzung im PMU in Abhängigkeit von der jahreszeitlich wechselnden Art der aufgenommenen Nahrung der trächtigen Stuten variieren kann, eliminiert werden, und damit die erhaltenen Extrakte alle einen vergleichbaren Gehalt konjugierter equiner Östrogene bezogen auf die Trockensubstanz aufweisen. Weiterhin kann eine Abtrennung der nicht-konjugierten lipophilen Verbindungen vorteilhaft sein, um ein gleichmäßiges physiologisches Wirkungsspektrum zu erzielen. Beispielsweise kann es sinnvoll sein, gegebenenfalls anwesende, nicht-konjugierte lipophile Verbindungen, die möglicherweise selbst eine eigene physiologische Wirkung besitzen können, aus dem natürlichen Gemisch konjugierter equiner Östrogene abzutrennen.

Eine Möglichkeit, um die unerwünschte, nicht-konjugierten lipophilen Verbindungen abzutrennen, wäre beispielsweise, die mit Hilfe der bekannten, oben aufgeführten Festphasen-Extraktionsverfahren gewonnenen natürlichen Gemische konjugierter equiner Östrogene einer separaten Flüssig-Flüssig-Extraktion mit einem geeigneten organischen Lösungsmittel zu unterziehen, welches gezielt die unerwünschten, nicht-konjugierten lipophilen Verbindungen extrahiert, ohne zu einem Verlust an konjugierten equinen Östrogenen zu führen. Ein solches Verfahren wird generell in der in der anhängigen internationalen Patentanmeldung PCT/EP 03/50703 beschrieben.

Die vorliegende Erfindung hat sich zum Ziel gesetzt, ein technisch und wirtschaftlich optimales Verfahren zur Gewinnung eines natürlichen Gemisches konjugierter equiner Östrogene zu entwickeln, wobei das Gemisch sowohl an phenolischen Harninhaltstoffen als auch an nicht-konjugierten lipophilen Verbindungen, insbesondere an nicht-konjugierten Flavonoid-, Isoflavonoid- und Norisoprenoid-Derivaten weitgehend abgereichert ist. Insbesondere ist es die Aufgabe der vorliegenden Erfindung ein Verfahren zu entwickeln, das im Vergleich zu den bereits bekannten Verfahren zur Gewinnung natürlicher Gemische konjugierter equiner Östrogene keine zusätzlichen Arbeitsschritte erfordert. Aufgabe der vorliegenden Erfindung ist die Entwicklung eines Verfahrens bei dem die Abreicherung sowohl an phenolischen Harninhaltstoffe als auch an nicht-konjugierten lipophilen Verbindungen während einer Festphasen-Extraktion erfolgt. Weiterhin soll ein Verfahren entwickelt werden, das nur auf wenigen Verfahrensschritten beruht und dabei einen Extrakt konjugierter equiner Östrogene liefert, welcher einen vergleichsweise hohen Gehalt an konjugierten Östrogenen bezogen auf die Trockensubstanz aufweist. Weiterhin soll es mit dem Verfahren der vorliegenden Erfindung auf einfache Weise möglich sein, auch dann ein natürliches Gemisch konjugierter Östrogene aus dem Harn trächtiger Stuten zu gewinnen, wenn der Harn wechselnde und gegebenenfalls erhöhte Mengen an nicht-konjugierten lipophilen Verbindungen enthält. Aufgabe der vorliegenden Erfindung ist es, ein optimiertes Verfahren zur Festphasen-Extraktion zu entwickeln, so dass das gewonnene natürliche Gemisch konjugierter equiner Östrogene gute Wirkstoffgehalte aufweist und die geforderten pharmazeutischen Spezifikationen erfüllt, insbesondere soll es die erforderlichen Grenzwerte hinsichtlich des Gehalts an konjugierten Östrogenen bezogen auf die Trockensubstanz einhalten.

Es wurde nun ein Verfahren gefunden, mit welchem auf überraschend einfache Weise ein an phenolischen Harninhaltsstoffen abgereichertes, weitgehend Kresol- und HPMF-freies Gemisch konjugierter equiner Östrogene aus PMU gewonnenen werden kann, welches gleichzeitig auch an nicht-konjugierten lipophilen Verbindungen, insbesondere an nicht-konjugierten Flavonoid-, Isoflavonoid- und Norisoprenoid-Derivaten weitgehend abgereichert ist, auch wenn der PMU wechselnde und gegebenenfalls erhöhte Mengen an nicht-konjugierten lipophilen Verbindungen aufweist.
Das erfindungsgemäße Verfahren basiert in wesentlichen Schritten auf dem in der WO 98/08526 beschriebenen Verfahren, welches zur Gewinnung eines an phenolischen Harninhaltsstoffen abgereicherten natürlichen Gemisches konjugierter Östrogene aus dem PMU dient. Weiterhin basiert das erfindungsgemäße Verfahren auf dem in der internationalen Patentanmeldung WO 03/048183 beschriebene n Verfa hren, mit dem auch bei Verwendung von gealtertem PMU, der gegebenenfalls erhöhte Anteile an freien Östrogenen enthält, ein natürliches Gemisch konjugierter Östrogene gewonnen werden kann, welches die geforderten pharmazeutischen Spezifikationen erfüllt

Demgemäß betrifft die Erfindung ein Verfahren zur Gewinnung eines natürlichen Gemisches konjugierter Östrogene aus dem Harn trächtiger Stuten, bei dem man
a) eine Harnflüssigkeit, welche gegebenenfalls eine von Schleim- und Feststoffen befreite Harnflüssigkeit, ein eingeengtes Konzentrat dieser Harnflüssigkeit oder ein durch Membranfiltration dieser Harnflüssigkeit erhaltenes eingeengtes Harnretentat darstellt, mit einer zur Adsorption des in der Harnflüssigkeit enthaltenen Gemisches konjugierter Östrogene ausreichenden Menge eines polymeren Adsorberharzes kontaktiert, und ein mit dem Gemisch konjugierter Östrogene beladenes polymeres Adsorberharz von der übrigen Harnflüssigkeit abtrennt, und
b) das mit dem Gemisch konjugierter Östrogene beladene polymere Adsorberharz mit einem auf einen pH-Wert von mindestens 12,0, insbesondere 12,5 bis 14,0, eingestellten Waschwasser wäscht, und
c) gegebenenfalls eine Zwischenwäsche durchführt, bei der das mit dem Gemisch konjugierter Östrogene beladene polymere Adsorberharz mit Wasser gewaschen wird, und
d) das gewaschene Adsorberharz mit einer zur Desorption des daran adsorbierten Gemisches konjugierter Östrogene ausreichenden Menge einer Elutionsflüssigkeit kontaktiert, und
e) ein das natürliche Gemisch konjugierter Östrogene enthaltende Eluat von dem Adsorberharz abtrennt und gegebenenfalls einengt,
wobei sich das erfindungsgemäße Verfahren gegenüber den Verfahren des Standes der Technik dadurch auszeichnet,
dass das gewonnene natürliche Gemisch konjugierter equiner Östrogene abgereichert ist an phenolischen Harninhaltsstoffen und an nicht-konjugierten lipophilen Verbindungen aus der Gruppe umfassend nicht-konjugierte Flavonoide, nicht-konjugierte Isoflavonoide, nicht-konjugierte Norisoprenoide, nicht-konjugierte Steroide, insbesondere Androstan- und Pregnan-Steroide, und an vergleichbaren nicht-konjugierten Verbindungen, und
dass man als Elutionsflüssigkeit im Verfahrensschritt (d) ein Ein- oder Zweiphasen-Gemisch, enthaltend
(i) Wasser, welches gegebenenfalls auf einen pH-Wert im alkalischen Bereich eingestellt ist, und
(ii) wenigstens ein für die Elution von nicht-konjugierten lipophilen Verbindungen aus oben angegebener Gruppe geeignetes, mit Wasser nicht oder kaum mischbares organisches Lösungsmittel,
   und gegebenenfalls
(iii) wenigstens ein mit Wasser mischbares organisches Lösungsmittel ausgewählt aus der Gruppe bestehend aus mit Wasser mischbaren Ethern, niederen Alkanolen und niederen aliphatischen Ketonen, sowie Mischungen der vorgenannten Lösungsmittel,
   verwendet, und
dass man, falls das im Verfahrensschritt e) erhaltene, gegebenenfalls eingeengte Eluat ein Zweiphasengemisch darstellt, die wässrige Phase des erhaltenen, aus zwei Phasen bestehenden Eluats abtrennt, und eine, das natürliche Gemisch konjugierter Östrogene enthaltende wässrige Phase gewinnt und diese gegebenenfalls einengt.

Die Chargenvorbereitung, die an sich bekannten Verfahrensschritte a), b), d) und e) sowie die Verwendung des im Verfahrensschritt e) erhaltenen Eluats, das ein Gemisch natürlicher konjugierter Östrogene enthält, sind generell bereits in der internationalen Patentanmeldung WO 98/08526 als Verfahrensschritte a), b) und c) beschrieben und dem Fachmann somit aus dieser publizierten Patentanmeldung geläufig. Der an sich bekannte Verfahrensschritt c) der vorliegenden Erfindung ist generell in der internationalen Patentanmeldung WO 03/048183 als "Zwischenwäsche" beschrieben und dem Fachmann somit aus dieser publizierten Patentanmeldung geläufig. Der Inhalt dieser WO 98/08526 sowie WO 03/048183 wird zum Zwecke der Offenbarung auch zum Gegenstand der vorliegenden Anmeldung gemacht. Weitere Einzelheiten zur allgemeinen Verfahrensweise und einsetzbaren Materialien sind im Beispielteil der vorliegenden Anmeldung zusammengefasst.

Beispielsweise können gemäß WO 98/08526 semipolare, insbesondere nichtionische semipolare Adsorberharze eingesetzt werden. Darüber hinaus ist es gemäß dem Verfahren der vorliegenden Erfindung überraschenderweise auch möglich andere Adsorberharze zusammen mit dem erfindungsgemäßen Verfahren einzusetzen, ohne dass die Produktqualität bzw. die einzuhaltende pharmazeutische Spezifikation beeinträchtigt wird. So sind im Rahmen der vorliegenden Erfindung polymere Adsorberharze für den Einsatz als Adsorbens für das erfindungsgemäße Verfahren geeignet. Die im Rahmen der vorliegenden Erfindung verwendbaren polymeren Adsorberharze werden im Beispielteil der Beschreibung noch näher erläutert.

Erfindungsgemäß wird mit dem vorliegenden Verfahren ein natürliches Gemisch konjugierter equiner Östrogene gewonnen, welches sowohl an phenolischen Harninhaltsstoffen wie Kresolen und HPMF als auch an nicht-konjugierten lipophilen Verbindungen aus der Gruppe umfassend nicht-konjugierte Flavonoide, nicht-konjugierte Isoflavonoide, nicht-konjugierte Norisoprenoide, nicht-konjugierte Steroide, insbesondere Androstan- und Pregnan-Steroide, und an vergleichbaren nicht-konjugierten Verbindungen, abgereichert ist.

Nach Durchführung der an sich bekannten Verfahrensschritte (a) bis (c) wird hierzu im Verfahrensschritt (d) das gewaschene, mit dem Gemisch konjugierter Östrogene beladene Adsorberharz mit einer zur Elution des Gemisches der konjugierten Östrogene ausreichenden Menge einer Elutionsflüssigkeit behandelt. Erfindungsgemäß wird hierzu als Elutionsflüssigkeit im Verfahrensschritt (d) ein Ein- oder Zweiphasen-Gemisch verwendet, welches
(i) Wasser, welches gegebenenfalls auf einen pH-Wert im alkalischen Bereich eingestellt ist,
   und
(ii) wenigstens ein für die Elution von nicht-konjugierten lipophilen Verbindungen aus der Gruppe umfassend nicht-konjugierte Flavonoide, nicht-konjugierte Isoflavonoide, nicht-konjugierte Norisoprenoide, nicht-konjugierte Steroide, insbesondere Androstan- und Pregnan-Steroide, und vergleichbare nicht-konjugierten Verbindungen, geeignetes, mit Wasser nicht oder kaum mischbares organisches Lösungsmittel,
   und gegebenenfalls
(iii) wenigstens ein mit Wasser mischbares organisches Lösungsmittel ausgewählt aus der Gruppe bestehend aus mit Wasser mischbaren Ethern, niederen Alkanolen und niederen aliphatischen Ketonen, sowie Mischungen der vorgenannten Lösungsmittel,
enthält.

In einer bevorzugten Ausführungsform der vorliegenden Erfindung wird als Elutionsflüssigkeit im Verfahrensschritt (d) ein Ein- oder Zweiphasen-Gemisch verwendet, welches
(i) Wasser, welches gegebenenfalls auf einen pH-Wert im alkalischen Bereich eingestellt ist,
   und
(ii) wenigstens ein für die Elution von nicht-konjugierten lipophilen Verbindungen aus der Gruppe umfassend nicht-konjugierte Flavonoide, nicht-konjugierte Isoflavonoide, nicht-konjugierte Norisoprenoide, nicht-konjugierte Steroide, insbesondere Androstan- und Pregnan-Steroide, und vergleichbare nicht-konjugierten Verbindungen, geeignetes, mit Wasser nicht oder kaum mischbares organisches Lösungsmittel,
   und
(iii) wenigstens ein mit Wasser mischbares organisches Lösungsmittel ausgewählt aus der Gruppe bestehend aus mit Wasser mischbaren Ethern, niederen Alkanolen und niederen aliphatischen Ketonen, sowie Mischungen der vorgenannten Lösungsmittel,
enthält.

Das als Elutionsflüssigkeit im Verfahrensschritt (d) Verwendung findende Ein- oder Zweiphasen-Gemisch enthält Wasser (i), welches gegebenenfalls auf einen pH-Wert im alkalischen Bereich eingestellt ist.

Handelt es sich bei der im Verfahrensschritt (d) Verwendung findenden Elutionsflüssigkeit um ein Einphasen-Gemisch, liegt der pH-Wert derartiger wasserhaltiger Elutionsflüssigkeiten im neutralen bis alkalischen Bereich bis zu pH 13 und kann vorteilhaft im Bereich zwischen pH 7 und 10 liegen. Als Lösungsmittelkomponenten in der wasserhaltigen Elutionsflüssigkeit werden in dem eingesetzten pH-Bereich stabile organische Lösungsmittel ausgewählt. Der gewünschte pH-Wert der wasserhaltigen, einphasigen Elutionsflüssigkeit wird durch Zugabe einer entsprechenden Menge einer wasserlöslichen inerten basischen Substanz, vorzugsweise einer anorganischen Base, beispielsweise eines Alkalimetall- oder Erdalkalimetallhydroxides, insbesondere Natriumhydroxid, eingestellt.

Handelt es sich bei der im Verfahrensschritt (d) Verwendung findenden Elutionsflüssigkeit um ein Zweiphasen-Gemisch, liegt der pH-Wert der wässrigen Phase derartiger Elutionsflüssigkeiten nach gründlicher Mischung beider Phasen im neutralen bis alkalischen Bereich bis zu pH 13 und kann vorteilhaft im Bereich zwischen pH 7 und 10 liegen. Sofern die wässrige Phase derartiger zweiphasiger Elutionsflüssigkeiten eine organische Lösungsmittelkomponente enthält, werden als Lösungsmittelkomponente in dem eingesetzten pH-Bereich stabile organische Lösungsmittel ausgewählt. Der gewünschte pH-Wert der wässrigen Phase von zweiphasigen Elutionsflüssigkeiten wird durch Zugabe einer entsprechenden Menge einer wasserlöslichen inerten basischen Substanz, vorzugsweise einer anorganischen Base, beispielsweise eines Alkalimetall- oder Erdalkalimetallhydroxides, insbesondere Natriumhydroxid, eingestellt.

Neben gegebenenfalls alkalisch eingestelltem Wasser (i) enthält das als Elutionsflüssigkeit im Verfahrensschritt (d) Verwendung findende Ein- oder Zweiphasen-Gemisch wenigstens ein, für die Elution von nicht-konjugierten lipophilen Verbindungen aus der Gruppe umfassend nicht-konjugierte Flavonoide, nicht-konjugierte Isoflavonoide, nicht-konjugierte Norisoprenoide, nicht-konjugierte Steroide, insbesondere Androstan- und Pregnan-Steroide, und vergleichbaren nicht -konjugierten Verbindungen geeignetes, organisches Lösungsmittel (ii). Weiterhin soll das derart geeignete organische Lösungsmittel (ii) mit Wasser nicht oder nur kaum mischbar sein, wobei kaum mischbar bedeutet, dass in der wässrigen Phase maximal 6 Vol.-% gelöstes organisches Lösungsmittels vorliegen. Im Rahmen des erfindungsgemäßen Verfahrens stellen für die Elution von nicht-konjugierten lipophilen Verbindungen geeignete, organische Lösungsmittel (ii) beispielsweise folgende organische Lösungsmittel mit 1 bis zu 10 C-Atomen dar, welche geradkettig, verzweigt oder zyklisch angeordnet sein können: C₄-C₁₀ Alkohole (wie beispielsweise Butanol, Hexanol, Cyclohexanol und Pentanol), C₁-C₁₀ veresterte Säuren (wie beispielsweise Ethylacetat (= Essigsäureethylester), Methylacetat, Propylacetat, Isopropylacetat (= Essigsäure-Isopropyl-Ester), Butylacetat, Amylacetat, Ethylmethylmalonat, Dimethylphosphonat), C₃-C₁₀ Aldehyde und C₄-C₁₀ Ketone (wie beispielsweise Butanon, Pentanon, Hexan-2,4-dion, Hexandial, Cyclohexancarbaldehyd, Butan-1,2,4-tricarbaldehyd, Methylphenylketon und ähnliche) bzw. generell C₃-C₁₀ Alkoxy-Verbindungen, C₃-C₁₀ Ether (Diethylether, Methyl-tertiär-butylether), C₃-C₆ Nitrilen, und C₁-C₃ Halogenalkanen (Methylenchlorid), sowie Mischungen der vorgenannten Lösungsmittel. Insbesondere können Essigsäure-C₁-C₄-alkylester, Butanol, Cyclohexanol, Hexanol, Diethylether, Methylenchlorid, Methyl-tertiär-butylether und Mischungen der vorgenannten Lösungsmittel als für die Elution von nicht-konjugierten lipophilen Verbindungen geeignete, mit Wasser nicht oder nur kaum mischbare organische Lösungsmittel (ii) im Rahmen der vorliegenden Erfindung genutzt werden. Aus dieser Auswahl stellen Essigsäure-C₁-C₄-alkylester, und insbesondere Essigsäureethylester (= Ethylacetat) und/oder Essigsäure -Isopropyl-Ester (= Isopropylacetat), die bevorzugt zu verwendenden, für die Elution von nicht-konjugierten lipophilen Verbindungen geeigneten, mit Wasser nicht oder nur kaum mischbaren organischen Lösungsmittel (ii) der vorliegenden Erfindung dar.

Weiterhin enthält das erfindungsgemäß als Elutionsflüssigkeit im Verfahrensschritt (d) Verwendung findende Ein- oder Zweiphasen-Gemisch gegebenenfalls wenigstens ein mit Wasser mischbares organisches Lösungsmittel (iii) ausgewählt aus der Gruppe bestehend aus mit Wasser mischbaren Ethern, niederen Alkanolen und niederen aliphatischen Ketonen, sowie Mischungen der vorgenannten Lösungsmittel. Als Etherbestandteile der Elutionsflüssigkeit eignen sich mit Wasser mischbare zyklische Ether wie Tetrahydrofuran oder Dioxan, aber auch mit Wasser mischbare offenkettige Ether wie beispielsweise Ethylenglycoldimethylether (= Monoglyme), Diethylenglycoldimethylether (= Diglyme) oder Ethyloxyethyloxyethanol (= Carbitol). Als niedere Alkanole eignen sich mit Wasser mischbare Alkylalkohole mit 1 - 4, vorzugsweise 1 - 3, Kohlenstoffatomen, insbesondere Ethanol oder Isopropanol. Als niedere aliphatische Ketone eignen sich mit Wasser mischbare Ketone mit 3 - 5 Kohlenstoffatomen, insbesondere Aceton. Als besonders günstig erweisen sich weiterhin Elutionsflüssigkeiten, in denen das mit Wasser mischbare organische Lösemittel (iii) Aceton, Ethanol oder eine Mischung aus Aceton und Ethanol ist.

In einer bevorzugten Ausgestaltung der Erfindung enthält das als Elutionsflüssigkeit im Verfahrensschritt (d) Verwendung findende Ein- oder Zweiphasen-Gemisch neben gegebenenfalls alkalisch eingestelltem Wasser (i) als für die Elution von nicht-konjugierten lipophilen Verbindungen geeignetes, mit Wasser nicht oder kaum mischbares organisches Lösungsmittel (ii) Essigsäure-C₁-C₄-alkylester, vorzugsweise Essigsäureethylester und/oder Essigsäure-Isopropyl-Ester, und als mit Wasser mischbares organisches Lösungsmittel (iii) Aceton und/oder Ethanol.

In einer weiteren bevorzugten Ausgestaltung der Erfindung enthält das als Elutionsflüssigkeit im Verfahrensschritt (d) Verwendung findende Ein- oder Zweiphasen-Gemisch neben gegebenenfalls alkalisch eingestelltem Wasser (i) als für die Elution von nicht-konjugierten lipophilen Verbindungen geeignetes, mit Wasser nicht oder kaum mischbares organisches Lösungsmittel (ii) Essigsäureethylester und als mit Wasser mischbares organisches Lösungsmittel (iii) Aceton.

Das als Elutionsflüssigkeit im Verfahrensschritt (d) erfindungsgemäß Verwendung findende Ein- oder Zweiphasen-Gemisch kann ein Volumenverhältnis von Wasser (i) zu dem mit Wasser nicht oder kaum mischbaren organischen Lösungsmittel (ii) im Bereich von 5:1 bis 1:5 aufweisen, insbesondere wird ein Volumenverhältnis im Bereich von 2:1 bis 1:2 als zweckmäßig angesehen.

Weiterhin kann das im Verfahrensschritt (d) als Elutionsflüssigkeit Verwendung findende Ein- oder Zweiphasen-Gemisch ein Volumenverhältnis von Wasser (i) zu dem mit Wasser mischbaren organischem Lösungsmittel (iii) im Bereich von 4:1 bis 1:5 aufweisen; vorzugsweise wird ein Volumenverhältnis im Bereich von 2:1 bis 1:3 als zweckmäßig angesehen.

Insbesondere kann das im Verfahrensschritt (d) als Elutionsflüssigkeit Verwendung findende Ein- oder Zweiphasen-Gemisch ein Volumenverhältnis von dem Gesamtvolumen an Wasser (i) zusammen mit dem mit Wasser nicht oder kaum mischbaren organischen Lösungsmittel (ii) im Verhältnis zu dem mit Wasser mischbaren organischem Lösungsmittel (iii) im Bereich von 5:1 bis 1:5, vorzugsweise im Bereich von 2:1 bis 1:2, aufweisen.

In einer besonders bevorzugten Ausgestaltungsform der vorliegenden Erfindung setzt sich das im Verfahrensschritt (d) als Elutionsflüssigkeit Verwendung findende Ein- oder Zweiphasen-Gemisch aus (i) Wasser, aus (ii) Essigsäure-C₁-C₄-alkylester, vorzugsweise Essigsäureethylester und/oder Essigsäure-Isopropyl-Ester, als mit Wasser nicht oder kaum mischbares organisches Lösungsmittel, und aus (iii) Aceton und/oder Ethanol als mit Wasser mischbaren organischem Lösungsmittel in einem Volumenverhältnis im Bereich von 1:1:1 bis 1:1:2 zusammen. Insbesondere findet im Verfahrensschritt (d) als Elutionsflüssigkeit ein Einphasen-Gemisch Verwendung, welches sich aus (i) Wasser, aus (ii) Essigsäureethylester als mit Wasser nicht oder kaum mischbares organisches Lösungsmittel, und aus (iii) Aceton als mit Wasser mischbaren organischem Lösungsmittel in einem Volumenverhältnis von 1:1:1,4 zusammensetzt.

Findet im Verfahrensschritt (d) als Elutionsflüssigkeit ein Zweiphasen-Gemisch Verwendung, empfiehlt es sich die zwei Phasen unmittelbar vor Aufgabe auf die Säule gut zu durchmischen.

Das eingesetzte Volumen an Elutionsflüssigkeit kann ca. 3 bis 10, insbesondere ca. 4 bis 6 Bettvolumen pro Bettvolumen polymeres Adsorberharz betragen. Zweckmäßigerweise wird die Elutionsflüssigkeit durch einen das mit dem Östrogengemisch beladene Adsorberharz enthaltenden Reaktor mit einer solchen Durchlaufgeschwindigkeit geleitet, dass die Kontaktzeit zur vollständigen Elution des Gemisches konjugierter Östrogene ausreicht. Bei Verwendung eines Einphasengemisches von Wasser, Essigsäureethylester und Aceton im Vol.-Verhältnis 10:10:14 eignen sich beispielsweise Durchlaufgeschwindigkeiten von 3 bis 10, vorzugsweise 5 bis 7, Vol.-Teilen Elutionsflüssigkeit pro 1 Vol.-Teil Adsorberharz pro Stunde. Zweckmäßig wird die Elution in einem Temperaturbereich von ca. 20°C bis ca. 80 °C, vorzugsweise bei Temperaturen zwischen ca. 30°C und 50°C, durchgeführt. Gewünschtenfalls wird die Durchlaufgeschwindigkeit durch Arbeiten bei leicht erhöhtem Druck, z. B. bei einem Überdruck von bis zu 0,2 bar, reguliert und das Eluat in mehreren Fraktionen aufgefangen.

Die Gehalte der einzelnen Eluatfraktionen an konjugierten Östrogenen, an phenolischen Harninhaltsstoffen wie Kresolen und HPMF und an nicht-konjugierten lipophilen Verbindungen können auf an sich bekannte Weise durch Hochleistungsflüssigkeits-Chromatographie (= high performance liquid chromatography, abgekürzt als "HPLC") oder auch Gaschromatographisch (abgekürzt als "GC") bestimmt werden.

Bei der Elution wird im Verfahrensschritt e) zunächst eine schwach gefärbte bis farblose, praktisch östrogenfreie Vorlauffraktion erhalten, deren Menge im Allgemeinen ca. einem Bettvolumen entspricht. Die Hauptmenge der konjugierten Östrogene, beispielsweise zwischen 80 und 99 % der in dem Ausgangs-PMU vorliegenden konjugierten Östrogene, befindet sich in den nachfolgenden dunkelgelb-braun gefärbten Haupteluatfraktionen, deren Menge im allgemeinen 2 bis 4 Bettvolumen umfasst. In den nachfolgenden Nachlauffraktionen sind im Allgemeinen nur noch Spuren an konjugierten Östrogenen enthalten. Sofern Nachfolgefraktionen erhalten werden, welche noch einen Gehalt an konjugierten Östrogenen von über 10 Gew.-% bezogen auf Trockensubstanz und weniger als 0,6 Gew.-% bezogen auf Trockensubstanz an Kresolen und HPMF enthalten, können diese mit dem östrogenreichen Haupteluat zur Weiterverarbeitung vereinigt werden.

Bei dem im Verfahrensschritt (e) erhaltenen Eluat kann es sich um ein Ein- oder Zweiphasengemisch handeln. Auch bei Verwendung eines Einphasengemisches als Elutionsflüssigkeit kann durch die Verdrängung des vor der Elution auf der Adsorberharzsäule vorhandenen Wassers von der Säule und der damit erhaltenen Verschiebung der Volumenverhältnisse von wässriger und organischer Phase das Eluat aus zwei Phasen bestehen. Gewünschtenfalls kann das im Verfahrensschritt (e) erhaltene ein- oder zweiphasige Eluat auf an sich bekannte Weise weiter eingeengt werden. Insbesondere wird, falls das im Verfahrensschritt (e) erhaltene Eluat einphasig ist, das Eluat eingeengt, so dass vorzugsweise das mit Wasser mischbare organische Lösungsmittel (iii) abgetrennt wird, bis das eingeengte Eluat ein Zweiphasen-Gemisch darstellt.

Erfindungsgemäß wird, falls das im Verfahrensschritt (e) erhaltene, gegebenenfalls eingeengte Eluat ein Zweiphasengemisch darstellt, in einem Verfahrensschritt (f) die wässrige Phase des erhaltenen, aus zwei Phasen bestehenden Eluats abgetrennt, und eine, das natürliche Gemisch konjugierter Östrogene enthaltende wässrige Phase gewonnen. Hierzu lässt man das erhaltene, zweiphasige Eluat stehen, um eine Trennung der Phasen zu erreichen. Die Phasentrennung kann je nach den erhaltenen Volumina einen Zeitraum von 10 min bis zu 24 Stunde n einne hmen, vorzugsweise lässt man die Phasen 5 bis 10 h stehen. Wenn sich die wässrige Phase und die organische Phase voneinander separiert haben, wird die wässrige Phase abgetrennt und zur Weiterverwendung aufgehoben, während die organische Phase verworfen wird.

Nach der Trennung der organischen Phase von der wässrigen Phase wird im Verfahrensschritt (f) eine, das natürliche Gemisch konjugierter Östrogene enthaltende wässrige Phase gewonnen. Diese wässrige Phase enthält das im PMU auftretende natürlich Gemisch konjugierter Östrogene neben nur einem geringen Anteil des ursprünglich in dem PMU vorhandenen Gehaltes an phenolischen Harninhaltsstoffen, wie Kresole und HPMF, sowie neben nur einem äußerst geringen Anteil des, ursprünglich im PMU vorhandenen Gehaltes an nicht-konjugierten lipophilen Bestandteilen. Gewünschtenfalls kann diese wässrige Phase auf an sich bekannte Weise weiter eingeengt werden, um ein weitgehend von organischem Lösungsmittel befreites, zur Weiterverarbeitung geeignetes Konzentrat zu erhalten. So können beispielsweise aus der erhaltenen wässrigen Phase die noch vorhandenen Reste an organischem Lösungsmittel abdestilliert werden. Durch die Destillation kann auch der Trockensubstanzgehalt der wässrigen Extraktphase auf einen konkreten Wert, vorzugsweise auf einen Trockensubstanzgehalt im Bereich zwischen 5 und 15%, insbesondere auf einen Trockensubstanzgehalt von 9%, eingestellt werden. Im Anschluss kann zur Stabilisierung des gewonnenen natürlichen Gemisches konjugierter equiner Östrogene noch der pH-Wert der wässrigen Extraktlösung auf einen Wert im alkalischen Bereich, vorzugsweise im Bereich zwischen 8 und 13, vorzugsweise auf einen Wert zwischen 9 und 12, eingestellt werden. Zur Einstellung des pH-Wertes eignen sich üblicherweise für die pH-Wert Einstellung Verwendung findende Basen, beispielsweise 1 N NaOH oder Na₂CO₃.

Die erfindungsgemäß im Verfahrensschritt (f) gewonnene und gegebenenfalls noch weiter aufgearbeitete oder konzentrierte wässrige Phase kann als Ausgangsmaterial für die Herstellung von, das natürliche Gemisch konjugierter equiner Östrogene enthaltenden Arzneimitteln dienen. Gewünschtenfalls kann durch einen geeigneten Trocknungsprozess, wie beispielsweise Sprühtrocknung oder Wirbelschichttrocknung, ein elutionsmittelfreies Feststoffgemisch hergestellt werden. Sofern das natürliche Gemisch konjugierter Östrogene für die Herstellung fester Arzneimittel verwendet werden soll, kann es zweckmäßig sein, der die konjugierten Östrogene enthaltenden wässrigen Phase einen festen Trägerstoff bereits vor einer Konzentrierung oder Trocknung zuzumischen, um auf diese Weise ein die konjugierten Östrogene und Trägerstoffe enthaltendes Feststoffgemisch zu erhalten. Beispielsweise kann die, die konjugierten Östrogene enthaltende wässrige Phase in der Wirbelschicht auf einen festen Trägerstoff wie z. B. Cellulose aufgesprüht werden. Sowohl die das Östrogengemisch enthaltende wässrige Phase als auch ein daraus hergestelltes Konzentrat oder getrocknetes Feststoffprodukt können in an sich bekannter Weise in feste oder flüssige galenische Zubereitungen wie beispielsweise Tabletten, Dragees, Kapseln oder Emulsionen eingearbeitet werden. Diese galenischen Zubereitungen können nach an sich bekannten Methoden hergestellt werden unter Verwendung üblicher fester oder flüssiger Trägerstoffe wie z. B. Stärke, Cellulose, Milchzucker oder Talkum oder flüssigen Paraffinen und/oder unter Verwendung von üblichen pharmazeutischen Hilfsstoffen, beispielsweise Tablettensprengmitteln, Lösungsvermittlern oder Konservierungsmitteln. So kann das die konjugierten Östrogene enthaltende Produkt mit den pharmazeutischen Träger- und Hilfsstoffen auf an sich bekannte Weise vermischt werden und das Gemisch in eine geeignete Dosierungsform überführt werden.

Bei dem erfindungsgemäß beschriebenen Festphasen-Extraktionsverfahren wird aus einer Harnflüssigkeit, welche gegebenenfalls eine von Schleim- und Feststoffen befreite Harnflüssigkeit, ein eingeengtes Konzentrat dieser Harnflüssigkeit oder ein durch Membranfiltration dieser Harnflüssigkeit erhaltenes eingeengtes Harnretentat darstellt, ein natürliches Gemisch konjugierter equiner Östrogene gewonnen, wobei das Gemisch in einem einfachen Verfahren sowohl an phenolischen Harninhaltstoffen wie insbesondere Kresolen und HPMF als auch an einer Vielzahl nicht-konjugierter lipophiler Verbindungen weitgehend abgereichert wird. Bei den abgetrennten nicht-konjugierten lipophilen Verbindungen handelt es sich insbesondere um nicht-konjugierte Flavonoide, nicht-konjugierte Isoflavonoide, nicht-konjugierte Norisoprenoide und nicht-konjugierte Steroide, hierbei insbesondere um nicht-konjugierte Androstan- und nicht-konjugierte Pregnan-Derivate.

Im Vergleich zu den bekannten Festphasen-Extraktionsverfahren liefert das erfindungsgemäße Festphasen-Extraktionsverfahren ein natürliches Gemisch konjugierter equiner Östrogene, welches sowohl an phenolischen Harninhaltstoffe als auch an nicht-konjugierten lipophilen Verbindungen weitestgehend abgereichert ist, wobei das erfindungsgemäße Verfahren ohne zusätzliche Arbeitsschritte auskommt, da auch die Abreicherung an nicht-konjugierten lipophilen Verbindungen bereits bei der Festphasenextraktion erfolgt.

Bei dem aus der WO 98/08526 bekannten Verfahren wird ein Teil der nicht-konjugierten lipophilen Verbindungen, beispielsweise das Isoflavon Equol, im Verfahrensschritt b) ausgewaschen, während andere nicht-konjugierte lipophile Verbindungen, beispielsweise Formononetin, mit in das Eluat gelangen und ggf. durch eine nachfolgende Flüssig-Flüssig-Extraktion abgetrennt werden müssen. Ähnliches gilt auch für die nicht-konjugierten, d.h. freien Steroidhormone, die sich ebenfalls zu gewissen Anteilen im Eluat nachweisen lassen. Es ist daher als ausgesprochen überraschend anzusehen, dass für die quantitative Elution der an ein polymeres Adsorberharz adsorbierten konjugierten Östrogene eine ein- oder sogar zweiphasige Mischung aus Wasser, einem mit Wasser nicht oder kaum mischbaren organischen Lösungsmittel, sowie ggf. einem mit Wasser mischbaren organischen Lösungsmittel wie niedere Ether, Alkanole oder Ketone, verwendet werden kann, und sich das erhaltene, ggf. eingeengte Eluat in zwei Phasen separieren lässt, wobei sich die - quantitativ eluierten - konjugierten Östrogene fast ausschließlich in der wässrigen Phase wieder finden, während sich die unerwünschten, nicht-konjugierten lipophilen Verbindungen in der organischen Phase befinden und somit leicht abgetrennt werden können.

Weiterhin liefert das erfindungsgemäße Verfahren einen Extrakt konjugierter equiner Östrogene, welcher einen vergleichsweise hohen Gehalt an konjugierten Östrogenen bezogen auf die Trockensubstanz aufweist. So ist es mit dem Verfahren der vorliegenden Erfindung auf einfache Weise möglich, auch dann ein natürliches Gemisch konjugierter Östrogene aus dem Harn trächtiger Stuten zu gewinnen, wenn der Harn wechselnde und gegebenenfalls erhöhte Mengen an nicht-konjugierten lipophilen Verbindungen enthält, ohne an die Festphasenextraktion weitergehende Aufreinigungsschritte anzuschließen.

Das durch das erfindungsgemäße Verfahren als Wirkstoff-Extrakt gewonnene, an nicht-konjugierten lipophilen Bestandteilen und an phenolischen Harninhaltstoffen abgereicherte natürliche Gemisch konjugierter equiner Östrogene zeichnet sich gegenüber den durch die bekannten Festphasen-Extraktionsverfahren gewonnenen Wirkstoff-Extrakte durch einen deutliche Optimierung der pharmazeutischen Spezifikation aus, wie erfindungsgemäß festgestellt wurde.

Es muss als ausgesprochen überraschend bezeichnet werden, dass durch die erfindungsgemäße Veränderung der bereits bekannten Festphasen-Extraktionsverfahren eine derartige Qualitätsverbesserung des gewonnenen Wirkstoffextraktes herbeigeführt werden kann, auch wenn der, das natürlichen Gemisches konjugierter Östrogene liefernde Harn trächtiger Stuten unterschiedliche und wechselnde Mengen nicht-konjugierte lipophiler Bestandteile enthält. Insbesondere ist es sehr überraschend, dass der Anteil an nicht-konjugierten lipophilen Verbindungen, der in Abhängigkeit vom verwendeten PMU sowohl mengenmäßig als auch in der Zusammensetzung stark schwanken kann, durch das erfindungsgemäße Verfahren derart sicher verringert werden kann, dass im Verfahrensschritt f) als wässrige Phase ein Gemisch natürlicher konjugierter equiner Östrogene erhalten werden kann, welches die hohen Anforderungen an die pharmazeutische Spezifikation, beispielsweise die gemäß der USP oder der Europäischen Pharmacopeia aufgestellten Anforderungen, erfüllt.

Das erfindungsgemäße Verfahren bietet, wie vorstehend bereits im Detail beschrieben, eine Reihe von Vorteilen und Verbesserungen gegenüber dem Stand der Technik. So ermöglicht die Erfindung die Verwendung auch von wechselnde Mengen nicht-konjugierter lipophiler Bestandteile enthaltendem PMU, der beispielsweise einen erhöhten Anteil an freien Flavonoiden, freien Isoflavonoiden, freien Norisoprenoiden oder freien Steroid-Derivaten, hier insbesondere an freien Androstan- oder Pregnan-Steroiden, aufweisen darf, ohne dass einzuhaltende pharmazeutische Spezifikationen gefährdet werden. Dabei baut das erfindungsgemäße Verfahren auf den bekannten Festphasenextraktionsverfahren auf, kommt aber überraschenderweise ohne weitere Extraktionsschrifte aus. So kann mit dem erfindungsgemäßen Verfahren eine gleichmäßige Zusammensetzung einzelner Extraktchargen gewährleistet werden, da die nicht-konjugierten lipophilen Bestandteile, deren Gehalt und Zusammensetzung im PMU in Abhängigkeit von der Art der aufgenommenen Nahrung der trächtigen Stuten variieren kann, stets eliminiert werden und damit die erhaltenen Extrakte alle einen vergleichbaren Gehalt konjugierter equiner Östrogene bezogen auf die Trockensubstanz aufweisen. Weiterhin wird durch die mit dem erfindungsgemäßen Verfahren erzielte Abtrennung nicht nur der phenolischen Haminhaltsstoffe, sondern insbesondere auch der nicht-konjugierten lipophilen Bestandteile in nur einem Arbeitsverfahren eine höhere Konzentration der Wirksubstanzen, d.h. der konjugierten equinen Östrogene, im erhaltenen Extrakt erreicht. Das erfindungsgemäße Verfahren weist zusätzlich auch wirtschaftliche Vorteile auf, da die Gefahr, wertvolle Wirkstoffe bei Nichteinhaltung der pharmazeutischen Spezifikation zu verlieren, beispielsweise bei nicht ausreichenden Gehalten konjugierter Östrogene bezogen auf die Trockensubstanz, deutlich vermindert ist. Weiterhin wird durch die Anwendung des erfindungsgemäß beschriebenen Verfahrens eine wesentlich genauere und reproduzierbarere Einstellung des Wirkstoffgehaltes des erhaltenen Extraktes ermöglicht. Diese Wirkstoffkomponente eignet sich hervorragend für die Herstellung von Pharmazeutika, die als Wirkstoff ein Gemisch natürlicher konjugierter equiner Östrogene enthalten.

Die nachfolgenden Beispiele sollen die Erfindung näher erläutern, ohne jedoch ihren Umfang zu beschränken.

### Beispiele

In nachfolgenden Beispielen wird eine allgemeine Arbeitsvorschrift zur Gewinnung von Wirkstoff-Extrakten aus PMU angegeben, die das natürliche Gemisch der im PMU enthaltenen konjugierten Östrogene enthalten, und sowohl an phenolischen HArninhaltsstoffen, wie beispielsweise Kresolen oder HPMF, als auch nicht-konjugierten lipophilen Verbindungen, wie beispielsweise nicht-konjugierte Flavonoide, nicht-konjugierte Isoflavonoide, nicht-konjugierte Norisoprenoide, nicht-konjugierte Steroide, insbesondere Androstan- und Pregnan-Steroide, und vergleichbare nicht-konjugierte Verbindungen, weitgehend abgereichert sind. Es wird gezeigt, wie sich erfindungsgemäß auch aus PMU, der wechselnde bzw. erhöhte Anteile an nicht-konjugierten lipophilen Verbindungen aufweisen kann, ein Qualitätsextrakt mit hohen Wirkstoffgehalten gewinnen lässt.

### Harnflüssigkeit:

Für das erfindungsgemäße Verfahren können als Harnflüssigkeit ebenso wie gemäß der WO 98/08526 der PMU als solcher, ein aus dem PMU durch Einengen gewonnenes Konzentrat oder ein aus dem PMU gewonnenes Konzentrat, welches durch Filtration oder vergleichbare Verfahren bereits vorgereinigt wurde, eingesetzt werden. Der gesammelte Harn (PMU) wird zunächst auf an sich bekannte Weise von Schleim- und Feststoffen befreit. Zweckmäßig lässt man Fest- und Schleimstoffe absetzen und trennt diese dann nach an sich bekannten Trennungsmethoden, beispielsweise Dekantieren, Separieren und/oder Filtrieren ab. So kann der PMU beispielsweise über eine an sich bekannte Trenneinrichtung, z. B. einen Separator, eine Filtrationsanlage oder einen Sedimentator geleitet werden. Als Trenneinrichtung kann z. B. ein Sandbett dienen, oder es können handelsübliche Separatoren, z. B. Düsen- oder Kammerseparatoren, eingesetzt werden. Gewünschtenfalls können auch eine Mikrofiltrationsanlage oder eine Ultrafiltrationsanlage eingesetzt werden, bei deren Verwendung gleichzeitig eine weitgehende Keim- und Virenfreiheit des filtrierten PMU erreicht werden kann.

Gegebenenfalls können dem Harn bzw. dem Harnkonzentrat Konservierungsmittel, Germizide, Bakterizide und/oder Antihelmintika zugesetzt werden.

Als vorgereinigtes Harnkonzentrat kann auch ein aufkonzentriertes PMU Retentat eingesetzt werden, welches aus dem PMU durch eine an sich bekannte Membranfiltration gewonnen werden kann. Der Feststoffgehalt des Retentates und dessen Zusammensetzung können je nach dem eingesetzten PMU und der zur Membranfiltration verwendeten Membran, beispielsweise deren Porenweite, sowie den Bedingungen der Filtration variieren. Beispielsweise kann bei Verwendung einer Nanofiltrationsmembran eine nahezu verlustfreie Konzentration des Östrogengehaltes in dem PMU Retentat bei gleichzeitiger Entfernung von bis zu 50 Gew.-% der niedermolekularen PMU Inhaltsstoffe erreicht werden. Für das erfindungsgemäße Verfahren können PMU Retentate eingesetzt werden, welche bis zu einem Verhältnis von ca. 1:10, beispielsweise einem Verhältnis von ca. 1:7 aufkonzentriert wurden und deren Volumen somit bis auf ca. 1/10, beispielsweise ca. 1/7, des ursprünglichen PMU Volumens eingeengt sein kann.

### Adsorberharze:

Die in dem Verfahrensschritt a) einsetzbaren polymeren Adsorberharze sind gemäß WO 98/08526 semipolare, insbesondere nicht-ionische semipolare, polymere Adsorberharze. Die im erfindungsgemäßen Verfahren als Adsorbens einsetzbaren polymeren Adsorberharze sind vorzugsweise poröse organische nichtionische Polymere, welche im Gegensatz zu unpolaren hydrophoben polymeren Adsorberharzen eine intermediäre Polarität (= z.B. mit einem Dipolmoment der aktiven Oberfläche des Harzes im Bereich von 1,0 bis 3,0, insbesondere 1,5 bis 2,0, Debye) und eine etwas hydrophilere Struktur besitzen, beispielsweise Polycarbonsäureesterharze. Zweckmäßig werden makroporöse semipolare Harze mit vorzugsweise makroretikulärer Struktur und mit durchschnittlichen Porendurchmessern im Bereich von 50 bis 150, vorzugsweise 70 bis 100, Angström und einer spezifischen Oberfläche im Bereich von 300 bis 900, vorzugsweise 400 bis 500, m²/g eingesetzt. Als besonders geeignet erweisen sich makroporöse quervernetzte aliphatische Polycarbonsäureesterharze, insbesondere quervernetzte Polyacrylesterharze wie z. B. Amberlite XAD-7 (Typ HP) der Fa. Rohm und Haas, die nicht-ionische semipolare Adsorberharze darstellen.

Neben den bevorzugt genannten Adsorbenzien können auch andere Adsorberharze eingesetzt werden. Als Adsorberharze eignen sich dabei sowohl unpolare, semipolare und auch polare Adsorberharze. Die Menge des Harns, die über den Adsorber gepumpt werden kann, ist hierbei zuvor anhand der jeweiligen Adsorberkapazität zu bestimmen. Beispiele für verwendbare Adsorberharze sind handelsübliche Typen wie polymere Amberlite Adsorbenzien mit Styroldivinylbenzolgrundgerüsten (z.B. Typen XAD-1180, XAD-2, XAD-16), mit Acrylestergrundgerüsten (z.B. XAD-7) oder solche mit hochpolaren Grundgerüsten enthaltend Stickstoff und Sauerstoff (z.B. XAD-12). Andere Adsorberharze sind Dowex-Harze (Copolymere von Styrol und Divinylbenzol), wie z.B. Dowex 112, Dowex Optipore, Dowex Optipore V 493; Lewatite (vernetzte Polystyrole), z.B. Lewatit OC 1064, Lewatit OC 1066 oder Lewatit OC 1163, Polyaminanionentauscherharze, z.B. Dowex-Harze. Vorteilhafte Adsorberharze sind insbesondere XAD-7 (Typ HP), XAD-16 (Typ HP), XAD 118 und Dowex Optipore, bevorzugt als Dowex Optipore V 493, sowie Lewatite OC 1064, OC 1066 und OC 1163.

### Verfahrensschritt a):

Die Adsorption der konjugierten Östrogene an das polymere Adsorberharz kann gemäß WO 98/08526 wie auch im vorliegenden erfindungsgemäßen Verfahren durch Kontaktierung des ggf. aufbereiteten PMU oder dessen Retentats mit dem Adsorberharz erfolgen, indem die Harnflüssigkeit in einen das Adsorberharz enthaltenden Reaktor eingeführt und darin für eine zur Adsorption des Östrogengehaltes ausreichende Zeit mit dem Adsorberharz in Kontakt gehalten wird. Nach erfolgter Adsorption der konjugierten Östrogene an das polymere Adsorberharz kann das mit dem Gemisch konjugierter Östrogene beladene Adsorberharz von der übrigen Harnflüssigkeit auf an sich bekannte Weise abgetrennt werden. Zweckmäßigerweise kann die Harnflüssigkeit durch eine das Adsorberharz enthaltende Säule mit solcher Durchlaufgeschwindigkeit geleitet werden, dass die Kontaktzeit zur Adsorption des Östrogengehaltes ausreicht. Geeignet sind beispielsweise Durchlaufgeschwindigkeiten, welche einem Durchlauf von 3 bis 10, vorzugsweise 5 bis 7, Vol.-Teilen PMU/1 Vol.-Teil Adsorberharz/Stunde entsprechen. Zweckmäßig kann die Durchlaufgeschwindigkeit der Harnflüssigkeit durch den Reaktor durch Arbeiten bei geringem Überdruck oder Unterdruck gesteuert werden. Die Menge an anzuwendendem polymeres Adsorberharz kann je nach Art des verwendeten Adsorberharzes und Menge des Feststoff-Gehaltes in der Harnflüssigkeit variieren. Bei Verwendung von PMU kann beispielsweise ein Volumenteil Adsorberharz, z. B. quervernetztes aliphatisches Polycarbonsäureester-Adsorberharz, mit bis zu 80, vorzugsweise von 30 bis 50, Volumen-Teilen vorbehandeltem PMU beladen werden, ohne dass merkliche Mengen Östrogen in der abfließenden Harnflüssigkeit nachweisbar sind. Bei Verwendung eines PMU-Konzentrates oder PMU-Retentates reduziert sich die Beladungskapazität des Adsorberharzes natürlich in dem Maße, in dem diese aufkonzentriert sind. So kann beispielsweise 1 Volumenteil an quervernetztem aliphatischem Polycarbonsäureester-Adsorberharz mit einer 20 bis 80, vorzugsweise 30 bis 50, Volumenteilen PMU entsprechenden Menge Harnflüssigkeit beladen werden.

### Verfahrensschritt b):

Das mit dem Gemisch konjugierter Östrogene beladene polymere Adsorberharz wird in Verfahrensschritt b) mit einem auf einen pH-Bereich von mindestens 12,0, insbesondere von 12,5 bis 14, vorzugsweise ca. 12,5 bis 13,5, eingestellten Waschwasser gewaschen. Als Waschwasser können wässrige Lösungen von in der Harnflüssigkeit löslichen inerten basischen Substanzen, welche stark genug sind, um einen pH-Wert von mindestens 12,5 zu erreichen, eingesetzt werden. Als gegenüber dem polymeren Adsorberharz inerte, wasserlösliche basische Substanzen eignen sich vorzugsweise wasserlösliche anorganische Basen wie Alkalimetall- oder Erdalkalimetallhydroxide, insbesondere Natriumhydroxid. Zweckmäßig enthält das Waschwasser nur etwa diejenige Menge an basischen Substanzen, welche zum Erreichen des gewünschten pH-Wertes, vorzugsweise ca. pH 13, benötigt wird. Die Menge an Waschwasser wird so gewählt, dass sie ausreicht, um phenolische Harninhaltsstoffe weitgehend zu entfernen, ohne dass dabei nennenswerte Mengen konjugierter Östrogene mit ausgewaschen werden. Als zweckmäßig erweist sich beispielsweise die Verwendung von 2 bis 10, insbesondere 4 bis 6, Bettvolumen Waschflüssigkeit pro Bettvolumen Adsorberharz. Hierbei wird das Waschwasser zweckmäßigerweise durch einen das Adsorberharz enthaltenden Reaktor mit einer Durchflussgeschwindigkeit von 3 bis 10, vorzugsweise 5 bis 7, Vol.-Teilen Waschwasser /1 Vol.-Teil Adsorberharz/Stunde geleitet.

### Verfahrensschritt c) - Zwischenwäsche:

Das im Verfahrensschnitt a) mit dem Gemisch konjugierter Östrogene beladene polymere Adsorberharz wird in einer sich an den Verfahrensschritt b) anschließenden Zwischenwäsche mit Wasser gewaschen. Die Menge an Waschwasser wird so gewählt, dass das im abschließenden Verfahrensschritt e) erhaltene Eluat ein Gemisch konjugierter Östrogene aufweist, das die Anforderungen an einen Höchstgehalt an freien Östrogenen erfüllt und damit als Wirkstoffkomponente für Pharmazeutika verwendet werden kann. Als zweckmäßig erweist sich beispielsweise die Verwendung von 1 bis 8, vorzugsweise 1 bis 4, Bettvolumen Waschwasser pro Bettvolumen Adsorberharz. Hierbei wird das Waschwasser zweckmäßigerweise durch einen das Adsorberharz enthaltenden Reaktor mit einer Durchflussgeschwindigkeit von 3 bis 10, vorzugsweise 5 bis 7, Vol.-Teilen Waschwasser pro 1 Vol.-Teil Adsorberharz pro Stunde geleitet.

In einer vorteilhaften Ausgestaltung des erfindungsgemäßen Verfahrens wird die Zwischenwäsche bei Temperaturen unterhalb Raumtemperatur, insbesondere bei Temperaturen zwischen 0°C und 10°C, durchgeführt, da sich gezeigt hat, dass durch die zusätzliche Zwischenwäsche gegebenenfalls bedingte Hormon- bzw. Wirkstoffverluste deutlich vermindert werden können. Als Raumtemperatur wird üblicherweise die Umgebungstemperatur angesehen, z.B. eine Temperatur bezeichnet, die zwischen 20° und 30°C liegt. Sehr zweckmäßig ist es, das Verfahren bei Temperaturen von tatsächlich 0°C bzw. annähernd 0°C auszuführen. In der Praxis empfiehlt es sich daher bei Temperaturen von nahe oberhalb 0°C zu arbeiten und durch entsprechende Maßnahmen für die Einhaltung der genannten Temperaturbereiche zu sorgen. Hierzu können an sich übliche Maßnahmen zur Absenkung der Temperatur getroffen werden, z.B. der Einsatz gekühlter Reaktoren, gekühlter Materialien und/oder gekühlter Ausgangsstoffe wie PMU. Als Temperaturen von 0°C bzw. von annähernd 0°C kann unter praktischen Gesichtspunkten ein Temperaturbereich von 0°C bis etwa 5°C, insbesondere von 0°C bis etwa 3°C, betrachtet werden.

Um mögliche Hormonverluste während der Zwischenwäsche so gering wie möglich zu halten, wird gemäß dieser Variante das in der Zwischenwäsche und/oder auch das im Verfahrensschritt b) verwendete alkalisch gestellte Waschwasser auf Temperaturen unterhalb Raumtemperatur, insbesondere auf Temperaturen zwischen 0°C und 10°C, vorgekühlt sein. Weitere zweckmäßige bzw. bevorzugte Temperaturbereiche sind wie vorstehend angegeben Temperaturen von 0°C bis etwa 5°C, insbesondere von 0°C bis etwa 3°C. Bevorzugt wird bei Temperaturen von 0°C bzw. von annähernd 0°C gearbeitet, d.h. vorzugsweise ist das in der Zwischenwäsche und/oder auch das im Verfahrensschritt b) verwendete alkalisch gestellte Waschwasser auf Temperaturen nahe oberhalb 0°C vorgekühlt. Durch die Verwendung von gekühltem alkalisch gestelltem Waschwasser im Verfahrensschritt b) wird eine Art Vorkühlung oder Aufrechterhaltung der bereits erfolgten Kühlung des Adsorberharzes erreicht, z.B. um zu vermeiden, dass beim Einsatz von gekühlten Waschwasser für die Zwischenwäsche eine unerwünschte Wiedererwärmung desselben stattfindet. Bevorzugt werden daher die Zwischenwäsche und der vorhergehende Verfahrensschritt b) beide im selben Temperaturbereich durchgeführt, z.B. bei Temperaturen unterhalb Raumtemperatur, insbesondere bei Temperaturen zwischen 0 °C und 10 °C, oder vorzugsweise in den wie oben angegebenen Temperaturbereichen.

In der vorstehenden Variante der Erfindung, in der das Verfahren bei Temperaturen unterhalb Raumtemperatur ausgeführt wird, kann es wünschenswert sein, alle verwendeten Vorrichtungen wie Reaktoren zur Aufnahme des polymeren Adsorberharzes oder dieses bereits enthaltende Reaktoren und/oder den eingesetzten PMU entsprechend auf Temperaturen unterhalb Raumtemperatur, insbesondere auf Temperaturen zwischen 0°C und 10°C, oder auf die oben angegebenen vorzugsweise einzuhaltenden Temperaturbereiche vorgekühlt einzusetzen.

### Verfahrensschritte d), e) und f) der vorliegenden Erfindung:

Anschließend wird gemäß der Beschreibung im Verfahrensschritt d) der vorliegenden Erfindung das gewaschene mit dem Gemisch konjugierter Östrogene beladene Adsorberharz mit einer zur Elution des Gemisches der konjugierten Östrogene ausreichenden Menge einer Elutionsflüssigkeit behandelt und im Verfahrensschritt e) wird ein das natürliche Gemisch der konjugierten Östrogene des PMU enthaltendes Eluat gewonnen und ggf. eingeengt. Falls das erhaltene, ggf. eingeengte Eluat ein Zweiphasengemisch darstellt, wird in einem Verfahrensschritt f) die wässrige Phase des Eluates abgetrennt, so dass eine das natürliche Gemisch der konjugierten Östrogene des PMU enthaltende, wässrige Phase gewonnen wird, welche ggf. weiter eingeengt werden kann.

### Regenerierung der Adsorberharz-Säule

Zur Regenerierung wird die Säule zunächst unter Verwendung von jeweils 1 bis 4, insbesondere 2 bis 3, Bettvolumen eines auf pH 13 eingestellten, 50 % Ethanol enthaltenden Ethanol/Wasser-Gemisches pro Bettvolumen Adsorberharz, dann mit dem entsprechenden Volumen 10-%iger wässriger Natriumcitratlösung und schließlich mit dem entsprechenden Volumen destilliertem Wasser gewaschen. Die gesamte Regeneration erfolgt bei einer Temperatur von 40°C bis 45°C. Die Säule kann mehrmals, beispielsweise bis zu 40-mal, beladen und wieder regeneriert werden.

### Beispiel 1:

### Vergleichsbeispiel entsprechend Verfahren der WO 98/08526 und WO 03/048183

### Beispiele 2a, 2b und 3 (erfindungsgemäßes Verfahren)

### Beispiel 2a: Elution mit 50-vol.% Ethylacetat bei Raumtemperatur

### Beispiel 2b: Elution mit 50-vol.% Ethylacetat bei 45°C

### Beispiel 3: Elution mit einem einphasigen Ethylacetat-haltigen Elutionsgemisch bei 40°C

### a) Adsorption des Östrogengehaltes des PMU an ein semipolares Polyacrylester-Adsorberharz (für alle Beispiele)

Es wird eine Säule von 330 mm Höhe mit einem Durchmesser von 40 mm mit 200 ml eines in Wasser gequollenen semipolaren Polyacrylester-Adsorberharz (= Amberlite XAD-7 (Typ HP) der Fa. Rohm und Haas, Korngröße 0,3 bis 1,2 mm, Dipolmoment 1,8 Debye, durchschnittlicher Porendurchmesser 80 Angström, spezifische Oberfläche ca. 450 m²/g trocken) gefüllt. 7 I (= 35 Bettvolumen, im Folgenden abgekürzt als BV) eines durch eine Ultrafiltrationsanlage filtrierten PMU, dessen Gehalt an konjugierten Östrogenen, berechnet als Summe aus Östronsulfat, Equilinsulfat und 17 α-DH-Equilin, sowie an Kresol mittels HPLC bestimmt wurde (Werte siehe Tabelle I), werden bei Raumtemperatur mit einer Durchflussgeschwindigkeit von durchschnittlich 16,7 ml/min (= 5 BV/h) in Beispiel 1, von 40 ml/min (= 12 BV/h) in Beispiel 2a und von 24,4 ml/min (= 7,3 BV/h) in Beispiel 2b und 3 durch die Säule geleitet. Der Östrogen-gehalt des PMU ist vollständig auf der so beladenen semipolaren Adsorberharz-Säule adsorbiert. Die ablaufende Harnflüssigkeit wird mittels HPLC auf ihren Gehalt an konjugierten Östrogenen untersucht und erweist sich als praktisch östrogenfrei. Der Ablauf wird verworfen.

### b) Waschen der beladenen Adsorberharz-Säule (für alle Beispiele)

Die beladene Adsorberharz-Säule wird mit 1,0 l (= 5 BV) einer wässrigen Natriumhydroxidlösung mit einem pH-Wert von 13 gewaschen. Hierzu wird das alkalische Waschwasser mit einer Durchflussgeschwindigkeit von durchschnittlich 16,7 ml/min (= 5 BV/h) durch die Säule geleitet. Die ablaufende Waschflüssigkeit wird mittels HPLC auf ihren Gehalt an konjugierten Östrogenen und Kresol untersucht. Die Untersuchung zeigt, dass während der Waschphase weniger als 5% der gesamten auf die Säule aufgegebenen Östrogene ausgewaschen werden.

### c) Zwischenwäsche (für alle Beispiele)

Die beladene Adsorberharz-Säule wird mit 600 ml Wasser (= 3 BV) gewaschen. Hierzu wird das neutrale Waschwasser mit einer Durchflussgeschwindigkeit von durchschnittlich 16,7 ml/min (= 5 BV/h) durch die Säule geleitet. Die ablaufende Waschflüssigkeit wird mittels HPLC auf ihren Gehalt an konjugierten Östrogenen und Kresol untersucht. Die Untersuchung zeigt, dass während dieser Waschphase nur ein geringer Teil (maximal nur etwa einige Prozent) der gesamten auf die Säule aufgegebenen Östrogene ausgewaschen wird.

### d) und e) Desorption der konjugierten Östrogene von der gewaschenen Adsorberharz-Säule

### Beispiel 1 - Vergleichsbeispiel

1,1 l (= 5,5 BV) der Elutionsflüssigkeit (Ethanol/Wasser 30:70) werden mit einer Fliessgeschwindigkeit von durchschnittlich 8,2 ml/min (= 2,4 BV/h) durch die auf eine Temperatur von 45°C vorgeheizte Säule geleitet. Das ablaufende Eluat wird in 6 Fraktionen E 1 bis E 5 aufgefangen. Die Fraktionen betragen jeweils 200 ml (= 1 BV) bzw 100 ml (= 0,5 BV für E 6) und werden mittels HPLC auf ihren Gehalt an konjugierten Östrogenen und Kresol untersucht (Werte siehe Tabelle I).

Die ersten vier Fraktionen enthalten ca. 80 bis 98 % der gesamten auf der Säule adsorbierten Menge konjugierter Östrogene. Die letzten Fraktionen enthalten nur noch geringe Menge an konjugierten Östrogenen. Die, die Hauptmenge der konjugierten Östrogene enthaltenden Fraktionen stellen für eine galenische Weiterverarbeitung geeignete Extrakte dar.

### Beispiel 2:

### Beispiel 2a - Elution mit 50-vol.% Ethylacetat bei Raumtemperatur

### Beispiel 2b - Elution mit 50-vol.% Ethylacetat bei 45°C

900 ml (= 4,5 BV) der zweiphasigen Elutionsflüssigkeit (Ethylacetat-Wasser Gemisch im Verhältnis 50:50) werden mit einer Fliessgeschwindigkeit von durchschnittlich 16,7 ml/min (= 5,0 BV/h) durch die, auf die angegebene Elutionstemperturtemperierte Säule geleitet. Das ablaufende Eluat wird in 9 Fraktionen E 1 bis E 9 aufgefangen. Die Fraktionen betragen jeweils 100 ml (= 0,5 BV); ab der vierten Fraktion wird als Eluat ein Zweiphasen-Gemisch erhalten, welches - zwecks Analyse für jedes Eluat einzeln - in die wässrige und organische Phase separiert wird (E 4 bis E 9, jeweils LM für Lösungsmittel und W für Wässrige Phase). Alle Fraktionen werden mittels HPLC auf ihren Gehalt an konjugierten Östrogenen und Kresol untersucht (Werte siehe Tabelle I). Für einige Fraktionen wird auch der Gehalt an nicht-konjugierten lipophilen Verbindungen am Beispiel von Formononetin mittels GC bestimmt (Werte siehe Tabelle II).

Die ersten drei Fraktionen enthalten nur Spuren an Östrogenen. In den nachfolgenden wässrigen Phasen der Fraktionen 4 bis 8 sind dann ca. 80 bis 98 % der gesamten auf der Säule adsorbierten Menge konjugierter Östrogene enthalten. Die letzten Fraktionen enthalten nur noch geringe Mengen an Östrogenen. Die, die Hauptmenge der konjugierten Östrogene enthaltenden Fraktionen stellen für eine galenische Weiterverarbeitung geeignete Extrakte dar.

Zur Kontrolle der Vollständigkeit der erfindungsgemäßen Elution mit dem Ethylacetat-Wasser-Gemisch wurde im Anschluss an die Extraktion die bereits bekannte Extraktion mit dem einphasigen Ethanol-Wasser-Gemisch (30:70) angeschlossen.

Durch die nachfolgende ethanolische Elution werden im Beispiel 2a jedoch noch deutliche Mengen konjugierter Östrogene, entsprechend ca. 6,8% der aufgegebenen Menge, eluiert. Durch die Erhöhung der Elutionstemperatur auf 45°C konnten die Menge der noch auf der Säule verbleibenden konjugierten Östrogene, die erst durch die ethanolische Elution eluiert werden, auf unter 1% reduziert werden.

### Beispiel 3 - Elution mit einem einphasigen Ethylacetat-haltigen Elutionsgemisch bei 40°C

1,0 l (= 5 BV) der einphasigen Elutionsflüssigkeit (Ethylacetat-Wasser-Aceton Gemisch im Volumenverhältnis 1:1:1,4) werden mit einer Fliessgeschwindigkeit von durchschnittlich 16,7 ml/min (= 5,0 Bettvolumen pro Stunde) durch die auf 40°C temperierte Säule geleitet. Das ablaufende Eluat wird in 13 unterschiedlich großen Fraktionen aufgefangen. Die erste Fraktion betrug 100 ml (= 0,5 BV), die nachfolgenden 10 Fraktionen betrugen jeweils 50 ml (= 0,25 BV), während die letzten beiden Fraktionen mit jeweils 200 ml aufgefangen wurden. Nach der Verdrängung des Waschwassers aus der Säule treten überraschenderweise bereits im Eluat (ab Fraktion 4) zwei Phasen auf. Alle Fraktionen werden mittels HPLC auf ihren Gehalt an konjugierten Östrogenen und Kresol untersucht (Werte siehe Tabelle I).

Die erste Fraktion enthält nur Spuren an Östrogenen. In den nachfolgenden Fraktionen 2 bis 7 sind dann ca. 80 bis 98 % der gesamten auf der Säule adsorbierten Menge konjugierter Östrogene enthalten. Die letzten Fraktion en enth alten nur noch geringe Mengen an Östrogenen. Die, die Hauptmenge der konjugierten Östrogene enthaltenden Fraktionen stellen für eine galenische Weiterverarbeitung geeignete Extrakte dar.

Die Eluate E7 bis E13 haben sich erst nach ca. 24h in 2 Phasen getrennt. Die Eluate E1 bis E7 werden vereinigt und folgendermaßen aufgearbeitet: Die Eluate E1 - E7 werden im 500 ml Tropftrichter vereinigt und ergeben ca. 350 ml Lösung. Es erfolgt keine sofortige Phasentrennung, aber nach dem Stehenlassen über das Wochenende ist eine kleine organische Phase von ca. 10 ml oben im Tropftrichter sichtbar. Die Phasen werden mittels HPLC auf ihren Gehalt an konjugierten Östrogenen und Kresol untersucht. Nach Auftrennung der Phasen wird die wässrige Phase am Rotationsverdampfer bei 70°C bis zu einem TS von 8-10% eingeengt (hier 8,16% TS, Werte siehe Tabelle I).

### Regenerierung der Adsorberharz-Säule (für alle Beispiele)

Zur Regenerierung wird die Säule zunächst mit 400 ml eines auf pH 13 eingestellten 50 % Ethanol enthaltenden Ethanol/Wasser-Gemisches, dann mit 400 ml 10-%iger wässriger Natriumcitratlösung und schließlich mit 400 ml destilliertem Wasser gewaschen. Die gesamte Regeneration erfolgt bei einer Temperatur von 40°C bis 45°C. Die Säule kann mehrmals, beispielsweise bis zu 40-mal, beladen und wieder regeneriert werden.

Für die bei der Elution erhaltenen, die Hauptmenge der konjugierten Östrogene enthaltenden Fraktionen sind in der nachfolgenden Tabelle jeweils der TS-Gehalt in Gew.-% und die durch HPLC bestimmten Gehalte an konjugierten Östrogenen (CE berechnet als die Summe aus Natrium-Östronsulfat, Natrium-Equilinsulfat und 17 α-DH-Equilin) sowie an Kresol angegeben. Weiterhin wird die Ausbeute der Extraktion als Wert angeführt.

**Tabelle I: Vergleich der einzelnen Verfahren anhand der Zusammensetzung der die Hauptmenge der konjugierten Östrogene enthaltenden Fraktionen**

| | **Vol** | **TS** | **Kresol** | **17α-DH- Equ.** | **Equilin** | **Estron** | **CE** | | | |
|---|---|---|---|---|---|---|---|---|---|---|
| | **[I]** | **Gew. %** | **[mg/l]** | **[mg/l]** | **[mg/l]** | **[mg/l]** | **[mg/l]** | **mg** | **Gew. %TS** | **Ausbeute** |
| **Beispiel 1 - Vergleichsverfahren** | | | | | | | | | | |
| **PMU** | 7,0 | n.b. | 329 | 25,3 | 57,9 | 64,9 | 148,1 | 1037 | n.b. | |
| **E 1** | 0,2 | 0,22 | 0,0 | 21,2 | 65,8 | 76,0 | 163,0 | 33 | 7,46 | |
| **E 2** | 0,2 | 1,08 | 0,0 | 345,9 | 1093,8 | 1237,0 | 2676,7 | 535 | 24,77 | |
| **E 3** | 0,2 | 0,44 | 0,0 | 183,4 | 524,7 | 607,8 | 1315,9 | 263 | 30,24 | |
| **E 4** | 0,2 | 0,10 | 0,0 | 30,2 | 58,2 | 84,6 | 173,0 | 35 | 16,99 | |
| **E 5** | 0,2 | 0,06 | 1,6 | 8,9 | 7,5 | 21,1 | 37,5 | 8 | 6,49 | |
| **E 2-4** * | 0,6 | 0,54 | 0,0 | 186,5 | 558,9 | 643,1 | 1388,5 | 833 | 25,71 | 80% |
| **Beispiel 2a - Elution mit Wasser /Ethylacetat 50:50 bei RT** | | | | | | | | | | |
| **PMU** | 7,0 | n.b. | 163,0 | 15,7 | 41,1 | 71,1 | 127,9 | 895 | n.b. | |
| **E 3** | 0,1 | 0,29 | 0,0 | 90,1 | 254,8 | 379,8 | 724,7 | 72 | 24,99 | |
| **E 4 W** | 0,064 | 2,70 | 266,0 | 735,5 | 1877,7 | 2858,2 | 5471,5 | 350 | 20,24 | |
| **E 5 W** | 0,053 | 1,00 | 188,8 | 466,9 | 1233,9 | 1877,4 | 3578,2 | 190 | 35,84 | |
| **E 6 W** | 0,051 | 0,50 | 98,9 | 245,6 | 666,9 | 1015,1 | 1927,6 | 98 | 38,68 | |
| **E 7 W** | 0,051 | 0,31 | 57,8 | 136,2 | 363,8 | 551,7 | 1051,7 | 54 | 33,82 | |
| **E 8 W** | 0,051 | 0,23 | 33,8 | 79,6 | 208,0 | 314,4 | 602,0 | 31 | 26,27 | |
| **E4-8*** | 0,27 | 1,02 | 136,1 | 353,1 | 921,3 | 1401,4 | 2675,8 | 722 | 25,91 | 81% |
| **Beispiel 2b - Elution mit Wasser /Ethylacetat 50:50 bei 45°C** | | | | | | | | | | |
| **PMU** | 7,0 | n.b. | 549,1 | 13,5 | 61,3 | 56,3 | 131,0 | 917 | n.b. | |
| **E 3** | 0,1 | n.b. | 31,8 | 26,6 | 124,4 | 122,7 | 273,7 | 27 | n.b. | |
| **E 4 W** | 0,086 | 2,67 | 0,0 | 401,9 | 1719,9 | 1684,2 | 3806,0 | 327 | 14,25 | |
| **E 5 W** | 0,058 | 1,49 | 0,0 | 519,8 | 2527,0 | 2268,4 | 5315,1 | 308 | 35,67 | |
| **E 6 W** | 0,058 | 0,60 | 0,0 | 227,1 | 878,3 | 884,1 | 1989,6 | 115 | 33,16 | |
| **E 7 W** | 0,056 | 0,25 | 0,0 | 84,9 | 350,7 | 357,9 | 793,6 | 44 | 31,74 | |
| **E 8 W** | 0,053 | 0,19 | 0,0 | 50,4 | 208,1 | 204,2 | 462,8 | 25 | 24,36 | |
| **E 4-8 *** | 0,311 | 1,21 | 0,0 | 274,3 | 1209,3 | 1152,9 | 2636,5 | 820 | 21,87 | 89% |
| **Beispiel 3 - Elution mit Wasser/Ethylacetat/Aceton 10:10:14 bei 40°C** | | | | | | | | | | |
| **PMU** | 7,0 | n.b. | 266,4 | 31,9 | 71,7 | 91,5 | 195,0 | 1365 | n.b. | |
| **E 1** | 0,1 | 0,30 | 0,0 | 58,4 | 126,2 | 180,8 | 365,3 | 37 | 12,18 | |
| **E 2** | 0,05 | 0,28 | 0,0 | 75,9 | 194,2 | 272,8 | 542,8 | 27 | 19,39 | |
| **E 3** | 0,05 | 1,40 | 0,0 | 794,0 | 1834,5 | 2304,5 | 4933,0 | 247 | 35,24 | |
| **E 4 W** | 0,046 | 4,24 | 0,0 | 2300,5 | 5446,5 | 6947,8 | 14694,8 | 676 | 34,66 | |
| **E 5 W** | 0,035 | 1,68 | 0,0 | 745,8 | 1722,8 | 2165,0 | 4633,5 | 162 | 27,58 | |
| **E 6 W** | 0,035 | 0,57 | 0,0 | 212,5 | 454,0 | 548,5 | 1215,0 | 43 | 21,32 | |
| **E 7** | 0,05 | 0,03 | 0,0 | 81,0 | 166,5 | 212,0 | 459,5 | 23 | - | |
| **E 8** | 0,05 | 0 | 0,0 | 9,6 | 20,1 | 25,0 | 54,7 | 3 | - | |
| **E1-7** * | 0,366 | 1,06 | 0,0 | 526,6 | 1227,0 | 1563,1 | 3316,8 | 1214 | 31,29 | 89% |
| **E1-7W** | 0,34 | 1,06 | 0,0 | 508,0 | 1146,6 | 1516,8 | 3171,4 | 1078 | 29,91 | 79% |
| **E1-7L** | 0,01 | n.b. | 0,0 | 97,0 | 235,0 | 340,0 | 672,0 | 7 | - | |
| **E1-7W Konz.** | 0,044 | 8,16 | 0,0 | 3878 | 8798 | 11780 | 24456 | 1076 | 29,97 | 79% |

| | | | | | | | | | | |
|---|---|---|---|---|---|---|---|---|---|---|
| *) rechnerische Bestimmung der Gehalte. | | | | | | | | | | |

Bei einer Versuchsdurchführung wie in Beispiel 1, d.h. bei einer ethanolischen Elution, wird ein Teil der Isoflavone, beispielsweise Equol, mit der basischen Wäsche ausgewaschen, ein Teil der Isoflavone, beispielsweise Formononetin, gelangt jedoch ins Eluat, und kann erst durch eine nachfolgende Extraktion abgetrennt werden. Ähnliches gilt für andere nicht-konjugierte lipophile Verbindungen, wie beispielsweise freie Steroidhormone. Demgegenüber gelangen bei dem erfindungsgemäßen Elutionsverfahren die noch auf der Säule befindlichen Isoflavone und freien Steroide zum überwiegenden Anteil in die, mit Wasser nicht-mischbare Lösungsmittelphase des ggf. eingeengten Eluates, während sich die konjugierten Östrogene zu ca. 98% in der weiter zu verarbeitenden wässrigen Phase dieses Eluates befinden. Diese wässrige Phase ist frei von nicht konjugierten Steroidhormonen und weitgehend abgereichert an Isoflavonen und vergleichbaren nicht-konjugierten lipophilen Verbindungen. Diese Verteilung wird anhand einer GC-Analyse der wässrigen Phase und der organischen Phase des 4. Eluates des Beispiels 2a verdeutlicht, wie nachfolgender Tabelle zu entnehmen ist.

**Tabelle II: Verteilung der konjugierten Östrogene, der freien Steroidhormone und des Formononetins (als Beispiel für Isoflavone) in der wässrigen Phase und der organischen Lösungsmittelphase des 4. Eluats des Beispiels 2a - Auswertung der GC-Analyse (die wässrige Phase wurde vor der Bestimmung ca. 1,8 fach konzentriert):**

| Eluat 4 | Masse | CE (gesamt) | | | Freie Hormone | | | Foromononetin | | |
|---|---|---|---|---|---|---|---|---|---|---|
| Phase | [g] | [mg/g] | [mg] | % Anteil | [mg/g] | [mg] | % Anteil | [mg/g] | [mg] | % Anteil |
| Wasser | 34,1 | 9,16 | 312 | 97,9 | 0 | 0 | 0 | 0,027 | 0,9 | 45,2 |
| Ethylacetat | 30,5 | 0,23 | 7 | 2,1 | 0,072 | 2,1 | 100 | 0,037 | 1,1 | 54,8 |

## Patentansprüche

1. Verfahren zur Gewinnung eines natürlichen Gemisches konjugierter Östrogene aus dem Harn trächtiger Stuten, bei dem man
a) eine Harnflüssigkeit, welche gegebenenfalls eine von Schleim- und Feststoffen befreite Harnflüssigkeit, ein eingeengtes Konzentrat dieser Harnflüssigkeit oder ein durch Membranfiltration dieser Harnflüssigkeit erhaltenes eingeengtes Harnretentat darstellt, mit einer zur Adsorption des in der Harnflüssigkeit enthaltenen Gemisches konjugierter Östrogene ausreichenden Menge eines polymeren Adsorberharzes kontaktiert, und ein mit dem Gemisch konjugierter Östrogene beladenes polymeres Adsorberharz von der übrigen Harnflüssigkeit abtrennt, und
b) das mit dem Gemisch konjugierter Östrogene beladene polymere Adsorberharz mit einem auf einen pH-Wert von mindestens 12,0 eingestellten Waschwasser wäscht, und
c) gegebenenfalls eine Zwischenwäsche durchführt, bei der das mit dem Gemisch konjugierter Östrogene beladene polymere Adsorberharz mit Wasser gewaschen wird, und
d) das gewaschene Adsorberharz mit einer zur Desorption des daran adsorbierten Gemisches konjugierter Östrogene ausreichenden Menge einer Elutionsflüssigkeit kontaktiert, und
e) ein das natürliche Gemisch konjugierter Östrogene enthaltende Eluat von dem Adsorberharz abtrennt und gegebenenfalls einengt,
**dadurch gekennzeichnet,**
**dass** das gewonnene natürliche Gemisch konjugierter equiner Östrogene abgereichert ist an phenolischen Harninhaltsstoffen und an nicht-konjugierten lipophilen Verbindungen aus der Gruppe umfassend nicht-konjugierte Flavonoide, nicht-konjugierte Isoflavonoide, nicht-konjugierte Norisoprenoide, nicht-konjugierte Steroide, insbesondere Androstan- und Pregnan-Steroide, und vergleichbare nicht-konjugierte Verbindungen, und
**dass** man als Elutionsflüssigkeit im Verfahrensschritt (d) ein Ein- oder Zweiphasen-Gemisch, enthaltend
(i) Wasser, welches gegebenenfalls auf einen pH-Wert im alkalischen Bereich eingestellt ist,
und
(ii) wenigstens ein für die Elution von nicht-konjugierten lipophilen Verbindungen aus oben angegebener Gruppe geeignetes, mit Wasser nicht oder kaum mischbares organisches Lösungsmittel,
und gegebenenfalls
(iii) wenigstens ein mit Wasser mischbares organisches Lösungsmittel ausgewählt aus der Gruppe bestehend aus mit Wasser mischbaren Ethern, niederen Alkanolen und niederen aliphatischen Ketonen, sowie Mischungen der
vorgenannten Lösungsmittel,
verwendet, und
**dass** man, falls das im Verfahrensschritt e) erhaltene, gegebenenfalls eingeengte Eluat ein Zweiphasengemisch darstellt, in einem Verfahrensschritt f) die wässrige Phase des erhaltenen, aus zwei Phasen bestehenden Eluats abtrennt, und eine, das natürliche Gemisch konjugierter Östrogene enthaltende wässrige Phase gewinnt und diese gegebenenfalls einengt.

2. Verfahren nach Anspruch 1, worin im Verfahrensschritt d) als Elutionsflüssigkeit ein Ein- oder Zweiphasen-Gemisch verwendet wird, welches
(i) Wasser, welches gegebenenfalls auf einen pH-Wert im alkalischen Bereich eingestellt ist, und
(ii) wenigstens ein für die Elution von nicht-konjugierten lipophilen Verbindungen aus der Gruppe umfassend nicht-konjugierte Flavonoide, nicht-konjugierte Isoflavonoide, nicht-konjugierte Norisoprenoide, nicht-konjugierte Steroide, insbesondere Androstan- und Pregnan-Steroide, und vergleichbare nicht-konjugierten Verbindungen, geeignetes, mit Wasser nicht oder kaum mischbares organisches Lösungsmittel, und
(iii) wenigstens ein mit Wasser mischbares organisches Lösungsmittel ausgewählt aus der Gruppe bestehend aus mit Wasser mischbaren Ethern, niederen Alkanolen und niederen aliphatischen Ketonen, sowie Mischungen der vorgenannten Lösungsmittel,
enthält.

3. Verfahren nach einem der vorherigen Ansprüche, worin das für die Elution von nicht-konjugierten lipophilen Verbindungen geeignete, mit Wasser nicht oder kaum mischbare organische Lösungsmittel (ii) ausgewählt ist aus der Gruppe bestehend aus geradkettigen, verzweigten oder zyklischen C₄-C₁₀ Alkoholen, C₁-C₁₀ veresterten Säuren, C₃-C₁₀ Aldehyden, C₄-C₁₀ Ketonen, C₃-C₁₀ Ethern, C₃-C₆ Nitrilen, und C₁-C₃ Halogenalkanen, sowie Mischungen der vorgenannten Lösungsmittel.

4. Ein Verfahren nach Anspruch 3, worin das für die Elution von nicht-konjugierten lipophilen Verbindungen geeignete, mit Wasser nicht oder kaum mischbare, organische Lösungsmittel (ii) ausgewählt ist aus der Gruppe bestehend aus Essigsäure-C₁-C₄-alkylester, Butanol, Cyclohexanol, Hexanol, Diethylether, Methylenchlorid, Methyl-tertiär-butylether und Mischungen der vorgenannten Lösungsmittel.

5. Ein Verfahren nach Anspruch 4, worin das für die Elution von nicht-konjugierten lipophilen Verbindungen geeignete, mit Wasser nicht oder kaum mischbare, organische Lösungsmittel (ii) Essigsäure-C₁-C₄-alkylester, vorzugsweise Essigsäureethylester und / oder Essigsäure-Isopropyl-Ester, ist.

6. Ein Verfahren nach einem der vorherigen Ansprüche, worin das mit Wasser mischbare organische Lösungsmittel (iii) ausgewählt ist aus der Gruppe bestehend aus Aceton, Ethanol und Tetrahydrofuran sowie Mischungen der vorgenannten Lösungsmittel.

7. Ein Verfahren nach Anspruch 6, worin das mit Wasser mischbare organische Lösungsmittel (iii) Aceton und/oder Ethanol ist.

8. Ein Verfahren nach Anspruch 7, worin das mit Wasser mischbare organische Lösungsmittel (iii) Aceton ist.

9. Ein Verfahren nach einem der Ansprüche 1 bis 4, worin das für die Elution von nicht-konjugierten lipophilen Verbindungen geeignete, mit Wasser nicht oder kaum mischbare, organische Lösungsmittel (ii) Essigsäure-C₁-C₄-alkylester, vorzugsweise Essigsäureethylester und/oder Essigsäure-Isopropyl-Ester, ist, und worin das mit Wasser mischbare organische Lösungsmittel (iii) Aceton und/oder Ethanol ist.

10. Ein Verfahren nach Anspruch 9, worin das für die Elution von nicht-konjugierten lipophilen Verbindungen geeignete, mit Wasser nicht oder kaum mischbare, organische Lösungsmittel (ii) Essigsäureethylester, und das mit Wasser mischbare organische Lösungsmittel (iii) Aceton ist.

11. Ein Verfahren nach einem der vorherigen Ansprüche, worin das im Verfahrensschritt (d) als Elutionsflüssigkeit Verwendung findende Ein- oder Zweiphasen-Gemisch ein Volumenverhältnis von Wasser (i) zu dem mit Wasser nicht oder kaum mischbaren, organischen Lösungsmittel (ii) im Bereich von 5:1 bis 1:5, vorzugsweise im Bereich von 2:1 bis 1:2, aufweist.

12. Ein Verfahren nach einem der vorherigen Ansprüche, worin das im Verfahrensschritt (d) als Elutionsflüssigkeit Verwendung findende Ein- oder Zweiphasen-Gemisch ein Volumenverhältnis von Wasser (i) zu dem mit Wasser mischbaren organischem Lösungsmittel (iii) im Bereich von 4:1 bis 1:5, vorzugsweise im Bereich von 2:1 bis 1:3, aufweist.

13. Ein Verfahren nach einem der vorherigen Ansprüche, worin das im Verfahrensschritt (d) als Elutionsflüssigkeit Verwendung findende Ein- oder Zweiphasen-Gemisch ein Volumenverhältnis von Wasser (i) und dem mit Wasser nicht oder kaum mischbaren, organischen Lösungsmittel (ii) zusammen zu dem mit Wasser mischbaren organischem Lösungsmittel (iii) im Bereich von 5:1 bis 1:5, vorzugsweise im Bereich von 2:1 bis 1:2, aufweist.

14. Ein Verfahren nach Anspruch 9, worin das im Verfahrensschritt (d) als Elutionsflüssigkeit Verwendung findende Ein- oder Zweiphasen-Gemisch ein Volumenverhältnis von Wasser (i) zu dem als mit Wasser nicht oder kaum mischbaren, organischen Lösungsmittel (ii) Verwendung findenden Essigsäure-C₁-C₄-alkylester, vorzugsweise Essigsäureethylester und/oder Essigsäure-Isopropyl-Ester, und zu dem als mit Wasser mischbaren organischem Lösungsmittel (iii) Verwendung findenden Aceton und/oder Ethanol im Bereich von 1:1:1 bis 1:1:2, vorzugsweise von etwa 1:1:1,4, aufweist.

15. Ein Verfahren nach einem der vorherigen Ansprüche, worin als polymere Adsorberharze semipolare Adsorberharze, vorzugsweise nicht-ionische semipolare Adsorberharze, verwendet werden.

16. Ein Verfahren nach einem der vorherigen Ansprüche, worin die Elution des natürlichen Gemisches konjugierter Östrogene vom gewaschenen Absorberharz im Verfahrensschritt d) bei Temperaturen im Bereich zwischen 20°C und 80°C, vorzugsweise bei Temperaturen zwischen 30°C und 50°C, durchgeführt wird.

## Claims

1. A method for obtaining a natural mixture of conjugated oestrogens from pregnant mares' urine, in which
a) a urine, which optionally represents a urine freed of mucilaginous substances and solids, a reduced concentrate of this urine or a reduced urine retentate obtained by membrane filtration of this urine, is contacted with an amount of a polymeric adsorption resin sufficient for the adsorption of the mixture of conjugated oestrogens contained in the urine, and a polymeric adsorption resin laden with the mixture of conjugated oestrogens is separated off from the rest of the urine, and
b) the polymeric adsorption resin laden with the mixture of conjugated oestrogens is washed with a washing water which has been set to a pH value of at least 12.0, and
c) optionally an intermediate washing operation is carried out, in which the polymeric adsorption resin laden with the mixture of conjugated oestrogens is washed with water, and
d) the washed adsorption resin is contacted with an amount of an elution liquid, sufficient for the desorption of the mixture of conjugated oestrogens adsorbed thereon, and
e) an eluate containing the natural mixture of conjugated oestrogens is separated off from the adsorption resin and optionally reduced,
**characterised in that**
the natural mixture of conjugated equine oestrogens obtained is depleted in phenolic urine contents and in non-conjugated lipophilic compounds from the group comprising non-conjugated flavonoids, non-conjugated isoflavonoids, non-conjugated norisoprenoids, non-conjugated steroids, in particular androstane and pregnane steroids, and comparable non-conjugated compounds, and
that a one- or two-phase mixture, containing
(i) water which is optionally set to a pH value in the alkaline range,
and
(ii) at least one organic solvent suitable for the elution of non-conjugated lipophilic compounds from the above group, which is not miscible, or only slightly miscible, with water,
and optionally
(iii) at least one water-miscible organic solvent selected from the group consisting of water-miscible ethers, lower alkanols and lower aliphatic ketones, and also mixtures of the aforementioned solvents,
is used as elution liquid in method step (d), and
that, if the optionally reduced eluate obtained in method step e) represents a two-phase mixture, in a method step f) the aqueous phase of the resulting eluate consisting of two phases is separated off, and an aqueous phase containing the natural mixture of conjugated oestrogens is obtained and optionally reduced.

2. A method according to Claim 1, wherein in method step d) a one- or two-phase mixture is used as elution liquid which contains
(i) water which is optionally set to a pH value in the alkaline range, and
(ii) at least one organic solvent suitable for the elution of non-conjugated lipophilic compounds from the group comprising non-conjugated flavonoids, non-conjugated isoflavonoids, non-conjugated norisoprenoids, non-conjugated steroids, in particular androstane and pregnane steroids, and comparable non-conjugated compounds which is not miscible, or only slightly miscible, with water, and
(iii) at least one water-miscible organic solvent selected from the group consisting of water-miscible ethers, lower alkanols and lower aliphatic ketones, and also mixtures of the aforementioned solvents.

3. A method according to one of the preceding claims, wherein the organic solvent (ii) suitable for the elution of non-conjugated lipophilic compounds which is not miscible, or only slightly miscible, with water is selected from the group consisting of straight-chain, branched or cyclic C₄-C₁₀ alcohols, C₁-C₁₀ esterified acids, C₃-C₁₀ aldehydes, C₄-C₁₀ ketones, C₃-C₁₀ ethers, C₃-C₆ nitriles and C₁-C₃ haloalkanes, and also mixtures of the aforementioned solvents.

4. A method according to Claim 3, wherein the organic solvent (ii) suitable for the elution of non-conjugated lipophilic compounds which is not miscible, or only slightly miscible, with water is selected from the group consisting of C₁-C₄-alkyl acetate, butanol, cyclohexanol, hexanol, diethyl ether, methylene chloride, methyl tert-butyl ether and mixtures of the aforementioned solvents.

5. A method according to Claim 4, wherein the organic solvent (ii) suitable for the elution of non-conjugated lipophilic compounds which is not miscible, or only slightly miscible, with water is C₁-C₄-alkyl acetate, preferably ethyl acetate and/or isopropyl acetate.

6. A method according to one of the preceding claims, wherein the water-miscible organic solvent (iii) is selected from the group consisting of acetone, ethanol and tetrahydrofuran and also mixtures of the aforementioned solvents.

7. A method according to Claim 6, wherein the water-miscible organic solvent (iii) is acetone and/or ethanol.

8. A method according to Claim 7, wherein the water-miscible organic solvent (iii) is acetone.

9. A method according to one of Claims 1 to 4, wherein the organic solvent (ii) suitable for the elution of non-conjugated lipophilic compounds which is not miscible, or only slightly miscible, with water is C₁-C₄-alkyl acetate, preferably ethyl acetate and/or isopropyl acetate, and wherein the water-miscible organic solvent (iii) is acetone and/or ethanol.

10. A method according to Claim 9, wherein the organic solvent (ii) suitable for the elution of non-conjugated lipophilic compounds which is not miscible, or only slightly miscible, with water is ethyl acetate, and the water-miscible organic solvent (iii) is acetone.

11. A method according to one of the preceding claims, wherein the one- or two-phase mixture used in method step (d) as elution liquid has a volume ratio of water (i) to the organic solvent (ii) which is not miscible, or only slightly miscible, with water in the range of 5:1 to 1:5, preferably in the range of 2:1 to 1:2.

12. A method according to one of the preceding claims, wherein the one- or two-phase mixture used in method step (d) as elution liquid has a volume ratio of water (i) to the water-miscible organic solvent (iii) in the range of 4:1 to 1:5, preferably in the range of 2:1 to 1:3.

13. A method according to one of the preceding claims, wherein the one- or two-phase mixture used in method step (d) as elution liquid has a volume ratio of water (i) and the organic solvent (ii) which is not miscible, or only slightly miscible, with water together to the water-miscible organic solvent (iii) in the range of 5:1 to 1:5, preferably in the range of 2:1 to 1:2.

14. A method according to Claim 9, wherein the one- or two-phase mixture used in method step (d) as elution liquid has a volume ratio of water (i) to the C₁-C₄-alkyl acetate, preferably ethyl acetate and/or isopropyl acetate, used as organic solvent (ii) which is not miscible, or only slightly miscible, with water, and to the acetone and/or ethanol used as water-miscible organic solvent (iii) in the range of 1:1:1 to 1:1:2, preferably of approximately 1:1:1.4.

15. A method according to one of the preceding claims, wherein semipolar adsorption resins, preferably non-ionic semipolar adsorption resins, are used as polymeric adsorption resins.

16. A method according to one of the preceding claims, wherein the elution of the natural mixture of conjugated oestrogens from the washed adsorption resin in method step d) is carried out at temperatures in the range of between 20°C and 80°C, preferably at temperatures between 30°C and 50°C.

## Revendications

1. Procédé d'obtention d'un mélange naturel d'oestrogènes conjugués à partir de l'urine de juments gravides, dans lequel
a) on met un liquide urinaire, qui est le cas échéant un liquide urinaire débarrassé des substances muqueuses et solides, un concentré réduit de ce liquide urinaire ou un rétentat urinaire concentré obtenu par filtration sur membrane de ce liquide urinaire, en contact avec une quantité d'une résine adsorbante polymère suffisante pour adsorber le mélange d'oestrogènes conjugués contenu dans le liquide urinaire et on sépare une résine adsorbante polymère chargée avec le mélange d'oestrogènes conjugués du reste du liquide urinaire ; et
b) on lave la résine adsorbante polymère chargée avec le mélange d'oestrogènes conjugués avec une eau de lavage ajustée à un pH au moins égal à 12,0 ; et
c) le cas échéant on effectue un lavage intermédiaire dans lequel la résine adsorbante polymère chargée avec le mélange d'oestrogènes conjugués est lavée avec de l'eau ; et
d) on met la résine adsorbante lavée en contact avec une quantité d'un liquide d'élution suffisante pour désorber le mélange d'oestrogènes conjugués adsorbé sur la résine ; et
e) on sépare un éluat contenant le mélange naturel d'oestrogènes conjugués d'avec la résine adsorbante et le cas échéant on le concentre,
**caractérisé en ce que** le mélange naturel d'oestrogènes équins conjugués ainsi obtenu est appauvri en constituants phénoliques de l'urine et en composés lipophiles non conjugués du groupe comprenant des flavonoïdes non conjugués, des isoflavonoïdes non conjugués, des norisoprénoïdes non conjugués, des stéroïdes non conjugués, en particulier des stéroïdes de l'androstane et du prégnane, et des composés non conjugués comparables, et **en ce que** l'on utilise comme liquide d'élution dans l'étape de procédé (d) un mélange monophasique ou diphasique contenant
(i) de l'eau, le cas échéant ajustée à un pH dans le domaine alcalin ; et
(ii) au moins un solvant organique non miscible ou peu miscible à l'eau approprié pour l'élution des composés lipophiles non conjugués du groupe précité ; et le cas échéant
(iii) au moins un solvant organique miscible à l'eau, choisi dans le groupe constitué par des éthers, des alcanols inférieurs et des cétones aliphatiques inférieures miscibles à l'eau, ainsi que des mélanges des solvants précités,
et **en ce que**, lorsque l'éluat obtenu dans l'étape de procédé e) représente un mélange diphasique le cas échéant concentré, on sépare dans une étape de procédé f) la phase aqueuse de l'éluat obtenu constitué de deux phases pour obtenir une phase aqueuse contenant le mélange naturel d'oestrogènes conjugués, laquelle est le cas échéant concentrée.

2. Procédé selon la revendication 1, dans lequel le liquide d'élution utilisé dans l'étape de procédé d) est un mélange monophasique ou diphasique contenant
(i) de l'eau, le cas échéant ajustée à un pH dans le domaine alcalin ; et
(ii) au moins un solvant organique non miscible ou peu miscible à l'eau approprié pour l'élution des composés lipophiles non conjugués du groupe comprenant des flavonoïdes non conjugués, des isoflavonoïdes non conjugués, des norisoprénoïdes non conjugués, des stéroïdes non conjugués, en particulier des stéroïdes de l'androstane et du prégnane, et des composés non conjugués comparables ; et
(iii) au moins un solvant organique miscible à l'eau, choisi dans le groupe constitué par des éthers, des alcanols inférieurs et des cétones aliphatiques inférieures miscibles à l'eau, ainsi que des mélanges des solvants précités.

3. Procédé selon l'une des revendications précédentes, dans lequel le solvant organique non miscible ou peu miscible à l'eau approprié pour l'élution des composés lipophiles non conjugués (ii) est choisi dans le groupe constitué par des alcools en C₄ à C₁₀ à chaîne droite, ramifiée ou cyclique, des acides en C₁ à C₁₀ estérifiés, des aldéhydes en C₃ à C₁₀, des cétones en C₄ à C_{10,} des éthers en C₃ à C₁₀, des nitriles en C₃ à C₆ et des haloalcanes en C₁ à C₃, ainsi que des mélanges des solvants précités.

4. Procédé selon la revendication 3, dans lequel le solvant organique non miscible ou peu miscible à l'eau (ii) approprié pour l'élution des composés lipophiles non conjugués est choisi dans le groupe constitué par des acétates d'alkyles en C₁ à C₄, le butanol, le cyclohexanol, l'hexanol, le diéthyléther, le chlorure de méthylène, le méthyl-*tert*-butyléther, ainsi que des mélanges des solvants précités.

5. Procédé selon la revendication 4, dans lequel le solvant organique non miscible ou peu miscible à l'eau (ii) approprié pour l'élution des composés lipophiles non conjugués est un acétate d'alkyle en C₁ à C₄, de préférence l'acétate d'éthyle et/ou l'acétate d'isopropyle.

6. Procédé selon l'une des revendications précédentes, dans lequel le solvant organique miscible à l'eau (iii) est choisi dans le groupe constitué par l'acétone, l'éthanol et le tétrahydrofurane, ainsi que des mélanges des solvants précités.

7. Procédé selon la revendication 6, dans lequel le solvant organique miscible à l'eau (iii) est l'acétone et/ou l'éthanol.

8. Procédé selon la revendication 7, dans lequel le solvant organique miscible à l'eau (iii) est l'acétone.

9. Procédé selon l'une des revendications 1 à 4, dans lequel le solvant organique non miscible ou peu miscible à l'eau (ii) approprié pour l'élution des composés lipophiles non conjugués est un acétate d'alkyle en C₁ à C₄, de préférence l'acétate d'éthyle et/ou l'acétate d'isopropyle, et dans lequel le solvant organique miscible à l'eau (iii) est l'acétone et/ou l'éthanol.

10. Procédé selon la revendication 9, dans lequel le solvant organique non miscible ou peu miscible à l'eau (ii) approprié pour l'élution des composés lipophiles non conjugués est l'acétate d'éthyle et le solvant organique miscible à l'eau (iii) est l'acétone.

11. Procédé selon l'une des revendications précédentes, dans lequel le mélange monophasique ou diphasique utilisé comme liquide d'élution dans l'étape de procédé (d) présente un ratio en volume de l'eau (i) sur le solvant organique non miscible ou peu miscible à l'eau (ii) dans la plage de 5:1 à 1:5, de préférence dans la plage de 2:1 à 1:2.

12. Procédé selon l'une des revendications précédentes, dans lequel le mélange monophasique ou diphasique utilisé comme liquide d'élution dans l'étape de procédé (d) présente un ratio en volume de l'eau (i) sur le solvant organique non miscible ou peu miscible à l'eau (ii) dans la plage de 4:1 à 1:5, de préférence dans la plage de 2:1 à 1:3.

13. Procédé selon l'une des revendications précédentes, dans lequel le mélange monophasique ou diphasique utilisé comme liquide d'élution dans l'étape de procédé (d) présente un ratio en volume de l'eau (i) sur le total du solvant organique non miscible ou peu miscible à l'eau (ii) et du solvant organique miscible à l'eau (iii) dans la plage de 5:1 à 1:5, de préférence dans la plage de 2:1 à 1:2.

14. Procédé selon la revendication 9, dans lequel le mélange monophasique ou diphasique utilisé comme liquide d'élution dans l'étape de procédé (d) présente un ratio en volume de l'eau (i) sur l'acétate d'alkyle en C₁ à C₄, de préférence l'acétate d'éthyle et/ou l'acétate d'isopropyle, utilisé comme solvant organique non miscible ou peu miscible à l'eau (ii) et l'acétone et/ou l'éthanol utilisé comme solvant organique miscible à l'eau (iii) dans la plage de 1:1:1 à 1:1:2, de préférence un ratio de 1:1 :1,4 environ.

15. Procédé selon l'une des revendications précédentes, dans lequel les résines adsorbantes polymères utilisées sont des résines adsorbantes semi-polaires, de préférence des résines adsorbantes semi-polaires non ioniques.

16. Procédé selon l'une des revendications précédentes, dans lequel l'élution du mélange naturel d'oestrogènes conjugués à partir de la résine adsorbante lavée dans l'étape de procédé d) se fait à des températures dans la plage de 20°C à 80°C, de préférence de 30°C à 50°C.
